# EUROPEAN PATENT SPECIFICATION

(11) **EP 3 846 867 B1**
(45) Date of publication and mention of the grant of the patent: **19.02.2025**
(21) Application number: 19765725.7
(22) Date of filing: 06.09.2019
(51) Int. Cl.: A61L 29/14, A61L 29/04, A61L 29/08, C08J 3/075, C08L 33/08

(54) **A MEDICAL TUBULAR DEVICE**
MEDIZINISCHE ROHRFÖRMIGE VORRICHTUNG
DISPOSITIF TUBULAIRE MÉDICAL

(30) Priority: 06.09.2018 DK PA201870574
(43) Date of publication of application: 14.07.2021
(73) Proprietor: Biomodics APS, 2800 Kgs. Lyngby (DK)
(72) Inventor: ALM, Martin, 2800 Lyngby (DK); THOMSEN, Peter, Theilade, 2800 Lyngby (DK)
(74) Representative: Plougmann Vingtoft a/s
(86) International application number: PCT/EP2019/073900
(87) International publication number: WO 2020/049177

(56) References cited:
- WO-A1-2016/183412
- WO-A1-2017/025104
- WO-A2-99/44665
- US-A1- 2008 181 861
- US-A1- 2008 181 861
- US-A1- 2010 040 870
- STENGER MICHAEL ET AL: "Co-release of dicloxacillin and thioridazine from catheter material containing an interpenetrating polymer network for inhibiting device-associatedStaphylococcus aureusinfection", JOURNAL OF CONTROLLED RELEASE, ELSEVIER, AMSTERDAM, NL, vol. 241, 20 September 2016 (2016-09-20), pages 125 - 134, XP029765687, ISSN: 0168-3659, DOI: 10.1016/J.JCONREL.2016.09.018

## Description

### TECHNICAL FIELD

The invention relates to a medical tubular device and a method for producing such medical tubular device. More specifically, the medical device disclosed herein is suitable for use in contact with a body fluid, such as blood. Example of medical tubular devices includes dialyse tubes, surgical drain tube and/or implants, such as implants comprising vascular grafts, dialysis grafts, central venous catheters, peripherally inserted central catheters, shunts, stents or stentgrafts etc.

### BACKGROUND ART

Medical tubular devices are essential devices within medical treatment of human and animal. Medical tubular devices are generally made of polymers, such as elastomeric polymers. Over the years a number of medical tubular devices of biocompatible materials for have been developed. In particular strength, flexibility, anti-microbial, non-fouling and/or non-thrombogenic characteristics are of importance.

US2006147665 discloses a vascular graft of expanded PTFE having a node and fibril microstructure.

US2006118236 discloses a layered prosthetic implantable device that offers a reduction in fluid loss when the device is punctured, such as by a dialysis needle or suture needle. The device includes inner and outer layers of a porous material having a microstructure of nodes interconnected by bent fibrils and having void spaces between adjacent bent fibrils. The inner and outer layers are joined by an elastomeric adhesive that may entangle to form an adhered polymer blended layer of the inner surface of the outer layer and the outer surface of the inner layer.

US2010/036476 discloses a method for reducing or preventing the occurrence of neointimal hyperplasia and/or thrombosis following implantation of a vascular implant, the method comprising, contacting a vascular implant with all-trans retinoic acid (ATRA); and implanting the vascular implant in a patient in need thereof, wherein the vascular implant comprises a biocompatible polymeric matrix; and the vascular implant releases a therapeutically effective amount of ATRA sufficient to inhibit or prevent neointimal hyperplasia and/or thrombosis when implanted in the patient.

US2015/238306 discloses a vascular graft comprising: a blood-contacting layer formed of a first microporous biomaterial; a nonporous intermediate layer; and a tissue-interface layer having a textured microporous surface that contacts host tissue when implanted.

US2016058913 discloses anti-thrombogenic vascular grafts, which are decellularized tissue coated with an anti-thrombogenic coating.

WO17066242 discloses an anti-microbial polymer composition for medical devices comprising anti-microbial oligomeric or polymeric additive. The additive includes bloom-promoting, adherence-promoting, and biocidally active monomers and/or moieties. The additive may further include a non-fouling and/or non-thrombogenic monomer and/or moieties.

STENGER ET AL (Journal of controlled release, vol.241, September 2016) disclose implantable catheters made of an interpenetrating polymer network.

WO 2017/025104 A1 discloses a drug release balloon made of an IPN. The host polymer is preferably a TPE. The guest (co)polymer can comprise zwitterionic monomers.

US 2008/181861 discloses an implantable medical device, tissue scaffold, membrane, contact lens or ship hull coated with a sulfobetaine or carboxybetaine material in an interpenetrating polymer network. The resulting surface is super low fouling. In one embodiment the sulfobetaine material is a copolymer comprising a polysulfobetaine domain and a poly(propylene oxide) domain.

### DISCLOSURE OF INVENTION

It is an objective of the invention to provide a new medical tubular device, which is very suitable for use in contact with body fluids, such as blood, urine, saliva or fragments thereof.

In an embodiment, it is an objective to provide a medical tubular device, which in use can maintain high patency for relatively long time and where the risk of occlusion is low.

In an embodiment, it is an objective to provide a medical tubular device, which has a desirable high suture retention strength.

In an embodiment, it is an objective to provide an implantable medical tubular device which when implanted has low risk of inducing neointimal hyperplasia and/or stenosis.

In an embodiment, it is an objective to provide a medical tubular device, which can be designed in a relatively simple way.

In an embodiment, it is an objective to provide a medical tubular device, which is highly suitable as a vascular graft and which, when implanted, has high patency for relatively long time and where the risk of occlusion is low.

In an embodiment, it is an objective to provide a method of producing the medical tubular device.

These and other objects have been solved by the invention as defined in the claims.

It has been found that the invention and embodiments thereof have a number of additional advantages, which will be clear to the skilled person from the following description.

The medical tubular device comprises a body structure having an elongate hollow structure extending from a first end to a second end of the medical tubular device and having a luminal surface and an external surface, wherein said body structure having a continuous matrix of a host polymer matrix and comprises an interpenetrating polymer network (IPN) comprising the host polymer matrix and at least one hydrogel guest polymer domain of a guest polymer comprising a plurality of interconnected paths of the guest polymer, which is interpenetrating the host polymer matrix, wherein said host polymer matrix is a covalently cross-linked elastomer matrix, wherein said at least one hydrogel polymer domain comprises a luminal surface guest polymer domain comprising at least a part of the luminal surface, wherein a plurality of the paths of the guest polymer coincide and form at least a part of said luminal surface and wherein said luminal surface comprises zwitterionic moieties, said zwitterionic moieties being covalently bonded to said guest polymer.

The medical tubular device of the invention has shown to be highly suitable for many different medical applications, as it will be described further below. The hydrogel domain(s) ensures a high compatibility in contact with tissue and further ensures that the medical tubular device may be designed for various purposes in a relatively simple way. The host polymer ensures that the medical tubular device has the desired mechanical properties. For example, the host polymer matrix may be selected to be very flexible. The host polymer matrix is an elastomer, e.g. a silicone elastomer, such that the medical tubular device practically mimics the mechanical properties of a native vessel. At the same time, the host polymer provides the medical tubular device with a high strength e.g. with a high suture retention strength suitably for being sutured to a native vessel. The elastic properties of the host polymer further may ensure that a wall part of the medical tubular device may be penetrated with a needle, where after when the needle is removed the penetrating hole is instantaneously closed (self-healing) due to the elastic properties. Thereby the medical tubular device may be highly suitable for use as a dialysis graft.

The term "biofluid" is herein used to mean a biological fluid, such as urine, sweat, saliva, breast milk, blood, cerebrospinal fluid, blister or cyst fluid.

The terms "moiety" and "moieties" are used interchangeable.

The phrase "suture retention strength" should be determined according to ISO7198:2016.

The term "substantially" should herein be taken to mean that ordinary product variances and tolerances are comprised.

The term "about" is generally used to include what is within measurement uncertainties. When used in ranges the term "about" should herein be taken to mean that what is within measurement uncertainties is included in the range.

The term "monomer" is herein used to denote single unit monomers and few unit monomers, also referred to as oligomers, such as oligomers comprising up to 10 monomeric units. In an embodiment, the monomer(s) is/are or comprises at least one single unit monomer.

In an embodiment, the monomer(s) is/are or comprises at least one oligomer.

The term "drug" may be a single chemical component or a composition or mixture of two or more components.

It should be emphasized that the term "comprises/comprising" when used herein is to be interpreted as an open term, i.e. it should be taken to specify the presence of specifically stated feature(s), such as element(s), unit(s), integer(s), step(s) component(s) and combination(s) thereof, but does not preclude the presence or addition of one or more other stated features.

Throughout the description or claims, the singular encompasses the plural unless otherwise specified or required by the context.

The "an embodiment" should be interpreted to include examples of the invention comprising the feature(s) of the mentioned embodiment.

The medical tubular device may in an embodiment be constituted by the body structure. In another embodiment, the medical tubular device may comprise additional element(s), such as supporting element(s), reinforcing element(s) and/or as described further herein.

The body structure advantageously has an elongate hollow structure, preferably with a generally round, preferably circular cross-sectional periphery. The body structure has a wall between the luminal and the external surface. The body structure may be straight, bended or branched and optionally with varying inner diameter, outer diameter and/or thickness along its length. In an embodiment, the body structure is tortuous, such as twisted.

The body structure may be bifurcated. It may be tapered or non-tapered (uniform).

The body structure comprises a continuous matrix of the host polymer and the at least one hydrogel polymer domain comprises a plurality of interconnected paths of the guest polymer. The at least one hydrogel polymer domain comprises a luminal surface guest polymer domain comprising at least a part of the luminal surface, an external surface guest polymer domain comprising at least a part of the external surface and/or an intermediate guest polymer domain which does not comprise the luminal surface or the external surface.

The hydrogel guest polymer is also referred to as the "guest polymer". The hydrogel guest polymer serves among other things to ensure a high flexibility and high biocompatibility of the body structure and thereby of the medical tubular device.

At least a part of the body structure wall is of the IPN. In an embodiment, substantially the entire body structure wall is of the IPN. In an embodiment, the body structure wall has a layered structure comprising two or more layers wherein at least one of the layers is of or comprises the IPN.

The host polymer matrix may be selected to ensure that the body structure has the desired mechanical properties, such as flexibility and/or strength, such as tear strength. The host polymer comprises a cross-linked elastomer, such as a thermoplastic elastomer (TPE), a polyolefin elastomer (POE), a polyurethane (PU), a rubber, thermoplastic polyurethane (TPU), a silicone elastomer or any combinations comprising one of the before mentioned elastomers.

It has been found that the medical tubular device wherein the host polymer is a cross-linked elastomer has a particular and desirable high suture retention strength. In addition, the medical tubular device with a cross-linked elastomer host polymer has shown to have a desirable low leak or risk of leak after repeated puncture, which makes it very suitable for use as a dialysis graft.

In an embodiment, the host polymer is a cross-linked polymer, such as a cross-linked elastomer e.g. thermoplastic elastomer (TPE), polyolefin elastomer (POE), polyurethane (PU), polyether block amide (PEBAX), rubber e.g. latex rubber, silicone or any combinations thereof.

The host polymer is covalently cross-linked. The covalently cross-linked host polymer is particularly desirable where high strength is required.

In an embodiment, the rubber is natural rubber or synthetic rubber, such as a vulcanized polymer of isoprene, the rubber is preferably a silicone rubber or cross-linked polyurethane.

Suitable TPUs include for example the TPUs marketed by Lubrizol under the tradenames Carbothane^{tm}, Isoplast^{®}, Pellethane^{®}, Tecoflex^{tm}, Tecophillic^{tm} and Tecothane^{tm}.

TPU is a linear segmented block copolymer composed of hard and soft segments. The hard segment can be either aromatic or aliphatic. Aromatic TPUs may be based on isocyanates such as MDI while aliphatic TPU's may be based on isocyanates like H12 MDI. When these isocyanates are combined with short-chain diols, they become the hard block. Normally it is aromatic, but when color and clarity retention in sunlight exposure is a priority, an aliphatic hard segment is often used.

TPU is relatively simple to process and may contain large amount of guest polymer. Further, TPU is highly elastic and has high resilience. The TPU comprises soft segments, which advantageously can be of polyether or polyester type. The TPU is preferably polyether-based.

In a preferred embodiment, the host polymer comprises silicone.

In an embodiment, the host polymer comprises at least 10 %, such as at least 20 %, such as at least 40 %, such as at least 60 % by mass of the host polymer with a backbone consisting of Si and O atoms or consisting of Si atoms, the host polymer preferably comprises poly(dimethyl siloxane), poly(methylphenyl siloxane), fluorosilicone rubber, silicone esters, polysiloxanes, polysilanes, polychlorosilanes, polyalkoxysilanes, polyaminosilanes, polysilanes, polydialkylsiloxanes, polysiloxanes containing at least one phenyl substituent, vinyl-functionalized silicone, partially or fully fluorinated silicone or a mixture of two or more of the mentioned silicones.

The guest polymer comprises one or more functional moieties.

The mechanical properties of the host polymer may be important in particular where the body structure consist of or mainly consist of the IPN because the hydrogel guest polymer may have poor mechanical strength and practically does not add to the strength of the medical tubular device. It has been found, that a high compliance and thus a low modulus add to ensure a high patency of the medical tubular device. However, a too low modulus may in some applications result in that the body structure is dilated up to an undesired diameter if the pressure in the medical tubular device is increased beyond a certain threshold. A too low modulus may result in a relatively low tear strength, which in some applications may be undesired. Further, in applications where the medical tubular device is adapted for suturing, the suturing may result in formation of needle penetration channels, which - in case of a high modulus - immediately will close, but in case of deformations that pull in the medical tubular device, may be re-opened and optionally cause undesired bleeding.

To meet one or more of these preferred properties, it has been found that the host polymer advantageously should have a stress at 200% elongation of at least about 0.3 MPa, such as at least about 0.4 MPa, such as at least about 0.5 MPa, such as at least about 0.6 MPa, such as up to about 3 MPa, such as up to about 2 MPa, such as up to about 1 MPa.

Advantageously, the host polymer has a Shore A hardness of from about 15 to about 70, such as from about 20 to about 55, such as from about 25 to about 50. Generally, a softer elastomer material will have a lower modulus than a less soft elastomer material.

Advantageously the host polymer has a tensile strength of at least about 8 MPa such as at least about 10 MPa, such as at least about 11 MPa or even higher e.g. 15 MPa and more.

Further, it is desired that the host polymer has a tear strength of at least about 25 kN/m, such as at least about 30 kN/m, such as at least about 35 kN/m, such as at least about 40 kN/m.

Examples of suitable host polymers include the Nusil^{™} Avantor^{™} High Consistency Silicone materials. In an embodiment, the host polymer is prepared with a surplus of residuals that may be extracted by an organic solvent and/or CO₂ (dense or supercritical) that swells the host polymer without dissolving it.

Examples of methods for providing IPNs are for example as described in the following publications:
Antimicrob. Agents Chemother 2017, 61(7), e00604-17 DOI:10.1128/AAC.00604-17. Plasmid 2016, 87-88, 72, DOI: 10.1016/j.plasmid.2016.10.001. J. Control. Release 2016, 241, 125-134 DOI: 10.1016/j.jconrel.2016.09.018. J. Mat. Chem. Phys. 2016, 181, 495-500 DOI: 10.1016/j.matchemphys.2016.06.086. J. Indus. Eng. Chem. 2016, 33, 142-149 DOI: 10.1016/j.jiec.2015.09.026. Biomacromolecules. 2016, 17(4), 1321-1329 DOI: 10.1021/acs.biomac.5b01722. J. Biomed. Mater. Res. Part B Appl. Biomater. 2016, 104(2), 402-410 DOI: 10.1002/jbm.b.33371. Eur. J. Pharm. Biopharm. 2015, 94, 305-311 DOI: 10.1016/j.ejpb.2015.05.014. Martin Alm, Peter Thomsen. A delivery device. Patent, PA 2015/70520. Martin Alm, Søren Langer Steffensen. A method of producing a delivery device. Patent, WO 2013/075724. Martin Alm, Maike Benter, Anne Marie Jensen. A method of producing an article comprising an interpenetrating polymer network (IPN) and an article comprising an IPN. Patent, WO 2008/052568. Martin Alm, Maike Benter, Anne Marie Jensen. A method of producing an article comprising an interpenetrating polymer network (IPN) and an article comprising an IPN. Patent, WO 2008/052563. Maike Benter, Martin Alm. A method of coating a polymer surface with a polymer containing coating and an item comprising a polymer coated polymer. Patent, WO 2006/074666. Joachim Karthauser, Maike Benter, and Martin Alm. A method of producing a silicone elastomer rubber item and the product obtainable by the method. Patent, WO 2006/045320.

The hydrogel guest polymer may be in swollen or in dry form, e.g. as a partly or fully swollen hydrogel, an aerogel or a xerogel. It may be desired to at least partially swell the hydrogel e.g. in aqueous media, such as water or saline water, prior to use. The amount of hydrogel polymer is generally given in dry form unless otherwise specified.

The body structure comprises a continuous matrix of the host polymer and a plurality of interconnected paths of the guest polymer. Advantageously the domain(s) of guest polymer forms a continuous interpenetrating pathway of the hydrogel guest polymer. Thereby the interpenetrating paths of the hydrogel may provide a passage for desired components and/or molecules and/or a passage for delivering a desired drug e.g. loaded into the hydrogel or otherwise deposited in contact with the hydrogel guest polymer. In an embodiment the drug is moulded into the host polymer e.g. during extrusion of the host polymer. The thereby embedded drug may be released via the interconnected paths of the guest polymer The luminal surface comprises a surface at least partly of the guest polymer i.e. at least one guest polymer domain comprises at least a part of the luminal surface. The guest polymer at the luminal surface may be the guest polymer at exit points of the interpenetrating paths and optionally it may include a coating comprising the guest polymer.

The surface coating of the guest polymer is considered to form a part of the at least one guest polymer domain when it is in contact with or linked to hydrogel guest polymer in a pathway of the host polymer. The surface coating is normally very thin since the guest polymer may have relative low mechanical strengths and in addition the layer may originate from propagating polymeric chains polymerizing (from within or) across the surface. A suitable surface coating may be e.g. less than 10 µm, such as about 1 µm or less, such as about 100 nm or less, such as about 50 nm or less, such as about 10 nm or less, e.g. as low as 1-2 nm.

The luminal surface with guest polymer has been found to be very compatible with the use in or in contact with a mammal body, e.g. where the medical tubular device is applied/utilized as a vascular graft, a dialysis graft or any other graft within the mammal body. It has been found that the medical tubular device ensures a very low risk of infection compared to prior art tubes for similar applications. Further, the risk of occlusion and low patency of the new medical tubular device is very low compared to prior art. In an embodiment, a plurality of the paths of the guest polymer coincides and form at least a part of the luminal surface.

In an embodiment, the guest polymer extends from a plurality of the paths to provide at least a part of the luminal surface.

In an embodiment, the luminal surface comprises a layer of a hydrogel polymer, such as a layer comprising or consisting of the guest polymer i.e. the same hydrogel polymer as the interpenetrating guest polymer.

In an embodiment, the luminal surface comprises a layer of a polymer, which is different from the guest polymer, such as another hydrogel polymer or a non-hydrogel. In an embodiment, the guest polymer may form cilia shaped formations (e.g. brushes and/or microscopic, hair-like structures) at the luminal surface and/or at the external surface, such as partly or fully entangled cilias shaped and/or bridge shaped strings of guest polymer.

Such cilia shaped formations and/or bridge shaped formations may e.g. be formed by subjecting the IPN with the guest polymer domain at a surface (luminal and/or external surface), to conditions switching in e.g. temperature and/or wettability (dry / wet / moist, cold / hot) a number of times, such as 5 times or more.

In an embodiment the external surface comprises a surface at least partly of the guest polymer, e.g. provided by a coating, the paths of the guest polymer coincide and form at least a part of the external surface and/or wherein the guest polymer extends from a plurality of the paths to provide at least a part of the external surface.

In an embodiment, the luminal surface comprises a layer of a polymer, which is different from the guest polymer, such as another hydrogel polymer or a non-hydrogel.

It should be understood that where it is disclosed that the guest polymer is located at the surface or any other location outside the host polymer, the guest polymer outside the host polymer is denoted "guest" to indicate that it is in contact with and/or linked to the guest polymer embedded and inside the host polymer.

In a variation thereof or in addition, the medical tubular device may comprise a hydrogel polymer coating which may not be in contact with or linked to a hydrogel guest polymer embedded and inside the host polymer. Such hydrogel polymer coating may be as the hydrogel guest polymer described herein with the difference that it is not in contact with or linked to a hydrogel guest polymer embedded and inside the host polymer.

The term "hydrogel polymer" as such may in an embodiment be interpreted to mean a hydrogel guest polymer (i.e. in contact with the interpenetrating guest polymer. The term "hydrogel polymer" as such may in another embodiment be interpreted to mean a hydrogel polymer not in contact with an interpenetrating guest polymer.

The external surface may advantageously be as described for the luminal surface above.

In an embodiment, the external surface comprises a layer of a polymer similar to or different from the guest polymer, such as a layer comprising or consisting of the guest polymer. Where the external surface comprises a layer of hydrogel polymer, the medical tubular device may be even further compatible with mammal tissue and thus highly suitable for use in contact with mammal tissue. As further described below the hydrogel polymer may comprise functional moieties, which may add further to the biocompatibility.

It is believed that the hydrophilic character of the hydrogel guest polymer domain at the luminal and/or the external surface provides a slippery surface, which reduces protein and bacterial adhesion while preserving normal immune response. It has been found that the luminal and/or the external surface mimics/resembles that of the native vessel and hence have a stealth character, preferably so that it does not trigger the compliment system and thereby reduce the risk of inflammation and/or infection. Furthermore, it has been found that the new medical tubular device has a very low risk of inducing neointimal hyperplasia and/or stenosis and/or ensures patency for an increased length of time.

It has been found that the hydrogel polymer e.g. provided in the form of the hydrogel guest polymer domain at the luminal and/or the external surface provides a highly desired non-fouling surface.

To further increase the biocompatibility, it has been found desirable that the luminal surface and/or the external surface comprise functional moieties in the form of zwitterionic moieties. The zwitterionic moieties are covalently bonded to the hydrogel (guest) polymer.

The term "hydrogel (guest) polymer" means the guest polymer and/or another hydrogel polymer at the luminal surface and/or the external surface of the body structure. The "another" hydrogel polymer may be as described for the guest polymer, however in the specific embodiment it may differ from the guest polymer of the guest polymer domain(s).

It has been found that zwitterionic moieties further increases the stealth character of the surface and thus reduce the risk of infection and/or inflammation. Where the medical tubular device is a vascular graft and the zwitterionic moieties are represented at the luminal surfaces, it has been found that the zwitterionic moieties ensure that the risk of thrombosis is reduced or even fully prevented. The zwitterionic moieties may additionally reduce or prevent activation of the compliment system, and coagulation cascade.

Thus, the luminal surface comprises zwitterionic moieties covalently bonded to the guest polymer.

In an embodiment, the external surface comprises zwitterionic moieties covalently bonded to the hydrogel (guest) polymer.

The zwitterionic moieties are provided by incorporating the zwitterionic moieties into the guest polymer. In an embodiment, the guest polymer comprises a zwitterionic hydrogel comprising a cross-linked network of polymerized monomers comprising one or more type of monomers, preferably comprising zwitterionic monomer(s) and/or a combination of cationic and anionic monomers.

A zwitterionic monomer is a monomer comprising at least one zwitterionic moiety. A cationic monomer is a monomer comprising at least one cationic moiety. An anionic monomer is a monomer comprising at least one anionic moiety. It should be noted that a monomer may be two or all three of the mentioned monomer types at the same time e.g. both an anionic monomer and a cationic monomer.

Examples of zwitterionic monomers include sulfobetanies, carbobetaines, phosphobetaines, phosphocholines or any combinations thereof.

In a preferred embodiment the zwitterionic monomer is or includes the zwitterionic monomer having the formula 1-Ethenyl-3-(4-sulfonatobutyl)-1*H*-imidazol-3-ium:

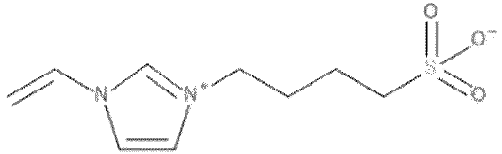

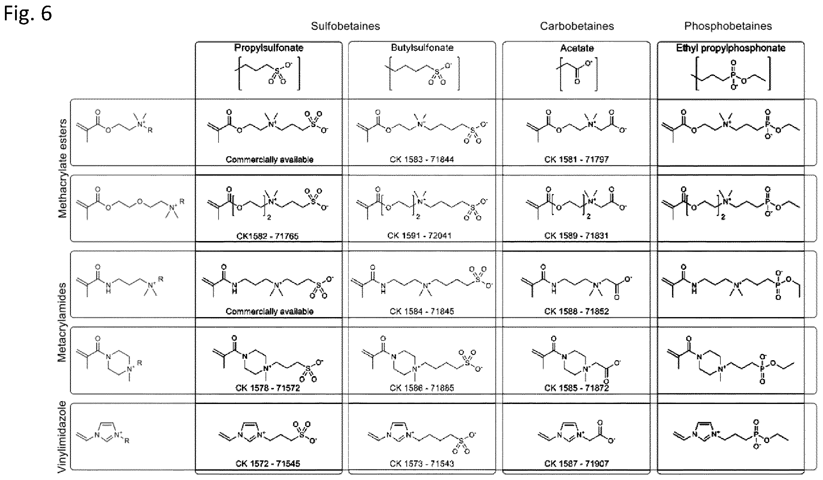

This is the zwitterionic monomer referred to on figure 6 as CK 1573.

In a preferred embodiment the zwitterionic monomer is or includes one or more of the zwitterionic monomers:
1-Ethenyl-3-(3-sulfonatopropyl)-1*H*-imidazol-3-ium (CK 1572): 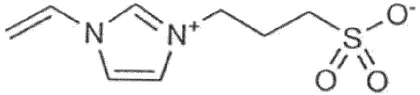
1-methyl-4-(2-methylprop-2-enoyl)-1-(4-sulfonatobutyl)piperazin-1-ium (CK1578): 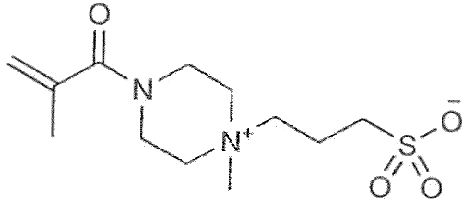
3-[dimethyl(2-{2-[(2-methylprop-2-enoyl)oxy]ethoxy}ethyl)azaniumyl]propane-1-sulfonate (CK1582): 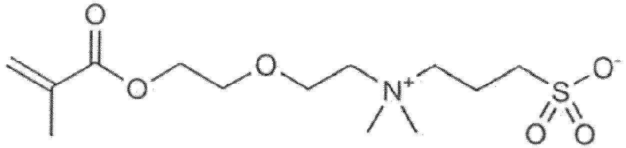
4-[dimethyl({2-[(2-methylprop-2-enoyl)oxy]ethyl})azaniumyl]butane-1-sulfonate (CK 1583):

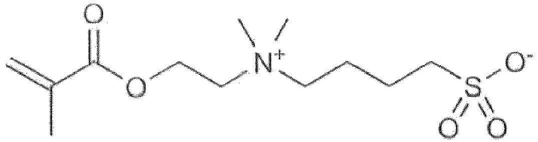

In a preferred embodiment the zwitterionic monomer is or includes one or more of the zwitterionic monomers:
4-(2-methylprop-2-enoyloxymethoxymethylammonio)butane-1-sulfonate (CK1591): 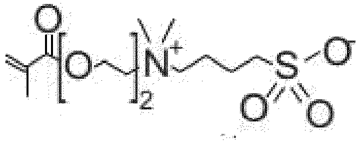
4-{dimethyl[3-(2-methylprop-2-enamido)propyl]azaniumyl} butane-1-sulfonate (CK 1584): 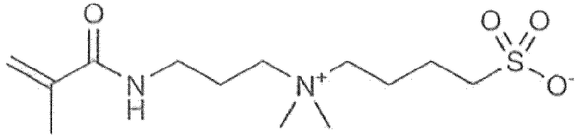
1-methyl-4-(2-methylprop-2-enoyl)-1-(4-sulfonatobutyl)piperazin-1-ium (CK 1586): 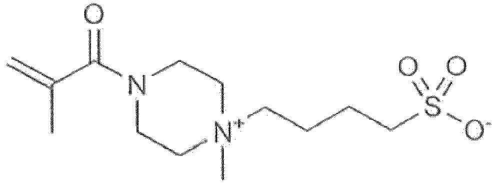
1-carboxy-N,N-dimethyl-N-(2'-methacryloyloxyethyl)methanaminium (CK 1581): 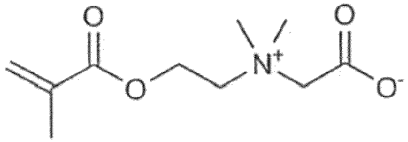
2-[dimethyl(2-{2-[(2-methylprop-2-enoyl)oxy]ethoxy}-ethyl)azaniumyl]acetate (CK 1589): 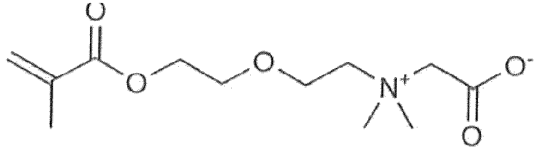
2-{dimethyl[3-(2-mehtylprop-2-enamido)propryl]-azamniumyl}acetate (CK 1588): 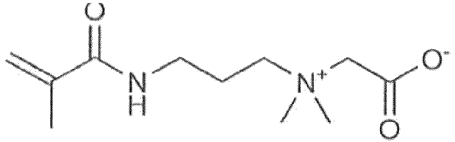
1-(carboxylatomethyl)-1-methyl-4-(2-methylprop-2-enoyl)piperazin-1-ium (CK 1585): 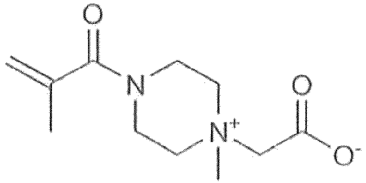
3-(carboxylatomethyl)-1-ethenyl-1H-imidazol-3-ium (CK 1587):

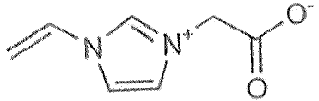

The IPN may further comprise one or more bonded drug(s), such as a drug at the luminal and/or the external surface. The drug may advantageously be covalent bonded to the hydrogel guest polymer. Advantageously the drug is specifically bonded to the guest polymer at the luminal and/or the external surface. In an embodiment, the body structure may comprise a first drug bonded to the hydrogel guest polymer at the external surface and a second, different drug bonded to the hydrogel (guest) polymer at the luminal surface.

Examples of drugs, which may be bonded, includes enzymes, immunosuppressive drugs, extracellular matrix proteins, antimicrobial, deactivating drugs and/or any combination comprising one of the mentioned drugs. In an embodiment, the drug that may be bonded is an anti-thrombogenic drug.

The drug is advantageously a drug having a catalytic function such that when it utilizes its biological effect, it is not spend but remain active over a desired period of time. Hence, when it is bonded at the luminal and/or external surface, the surface will have and substantially maintain the biological effect of the drug.

The drug may be selected in accordance with the intended application of the medical tubular device.

In an embodiment the body structure comprises at least one drug bonded (preferably covalently bonded) to the guest polymer and/or to the host polymer at the luminal surface.

In an embodiment the body structure comprises at least one drug bonded (preferably covalently bonded) to the guest polymer and/or to the host polymer at the external surface.

Advantageously the bonded (e.g. covalently bonded) drug is a thrombosis deactivating drug(s). The thrombosis deactivating drug(s) preferably comprises heparin, EDTA, antibiotics citrate and/or any combination comprising one of the mentioned thrombosis deactivating drugs.

Heparin is a well-known thrombosis deactivating drug. Preferably, heparin molecules are bound directly to the luminal surface body structure. Heparin is a polysaccharide anticoagulant having a potent anti proliferative effect on vascular smooth muscle cells. Heparin at the luminal surface has been found to effectively inactivate thrombin to thereby ensure that fibrin is not converted to insoluble strands but remain soluble and thereby the heparin reduces or eliminates the risk of thrombosis.

It is anticipated that the thrombosis deactivating effect of the thrombosis deactivating drug(s) may last for a very long time, such at more than 1, 2 or 3 year or even longer.

The covalently bonded drug may advantageously be covalently bonded to the hydrogel (guest) polymer and/or the host polymer via a linker molecule to ensure an optimal effect of the drug. Suitable linker includes the linkers described in US20080227092. Prior to being bonded to the drug, the linker advantageously comprises a functional moiety (e.g. capture moiety) for being bonded to the drug. The linker may e.g. be cleavable to expose the functional moiety. The body structure may in addition comprise one or more releasable drugs, e.g. to further improve its biocompatibility and beneficial effect(s).

In an embodiment, the releasable drug comprises an anti-proliferative drug, such as Paclitaxel (Taxol) and/or Rapamycin and/or other suitable anti-proliferative drugs.

Where there may be risk of cell growth inside the lumen of the medical tubular device, it is desired that the anti-proliferative drug is located at least as a part of the luminal surface.

Where it is desired that the medical tubular device should not grow together with the surrounding tissue it is in contact with in use, the anti-proliferative drug may advantageously be located at least as a part of the external surface of the body structure. Such, medical tubular device with low ingrowth properties may for example be a tube for temporarily use to be inserted in contact with tissue of a patient, e.g. a tube for supplying chemotherapy drugs to a patient. In addition, where the medical tubular device is suitable for implant and where it is anticipated that it may be required to retrieve or replace the implant, it may be desirable that it has a low ingrowth potential and hence, may be recovered or explanted in a relatively simple way.

In an embodiment, the releasable drug comprises an anti-infective drug, preferably selected from Rifampicin and/or minocycline.

The anti-infective drug is advantageously a drug that is capable of acting against infection, either by inhibiting the attachment and spread off an infectious agent or by killing the infectious microorganism outright.

The anti-infective drug may e.g. comprise antibiotic and/or antiseptic drug(s).

The anti-infective drug may in particularly be desired where there is high risk of infection, such as in situations where the medical tubular device is adapted for use in contact with a patient with high risk of infection and/or with low immune defence.

The anti-infective drug may be located anywhere in the medical tubular device. Advantageously the anti-infective drug is located at the external surface of the body structure to reduce risk to infection between the body structure and the tissue it is adapted to be in contact with. This may limit and/or reduce the risk of surgical site infections. Dialysis graft generally have higher risk of infection than "normal" grafts, since there is a considerable risk of introducing/inflicting an infection each time the patient receives treatment (dialysis) where needles penetrate the skin, tissue and graft (this might occur several times every week).

Blood access devices (such as CVC and dialysis grafts) have a high risk of infections. Especially central venous catheters (CVC graft) have a large risk of infection, because they provide a pathway for bacteria from outside to inside the body.

In an embodiment, the anti-infective drug is located in a guest polymer domain, which is located at a distance to both the luminal surface and the external surface i.e. in an intermediate guest polymer domain, which does not comprise the luminal surface or the external surface. In such case the anti-infective drug may be released upon penetrating the body structure e.g. by a needle during suturing or by a needle where the medical tubular device is a dialysis graft. Thus, the release of the anti-infective drug may be located to high-risk locations and further the release period may be very long until the releasable amount of anti-infective drug is used.

In an embodiment the anti-infective drug comprises one or more of amebicides; aminoglycosides; anthelmintics; antifungals, such as azole antifungals, echinocandins, miscellaneous antifungals or polyenes; antimalarial agents, such as antimalarial combinations, antimalarial quinolines or miscellaneous antimalarials; antituberculosis agents, such as aminosalicylates, antituberculosis combinations, diarylquinolines, hydrazide derivatives, miscellaneous antituberculosis agents, nicotinic acid derivatives, rifamycin derivatives or streptomyces derivatives; antiviral agents, such as adamantane antivirals, antiviral boosters, antiviral combinations, antiviral interferons, chemokine receptor antagonist, integrase strand transfer inhibitor, miscellaneous antivirals, neuraminidase inhibitors, NNRTIs, NS5A inhibitors, nucleoside reverse transcriptase inhibitors (NRTIs), protease inhibitors or purine nucleosides; carbapenems; carbapenems/beta-lactamase inhibitors; cephalosporins, such as cephalosporins/beta-lactamase inhibitors, first generation cephalosporins, fourth generation cephalosporins, next generation cephalosporins, second generation cephalosporins or third generation cephalosporins; glycopeptide antibiotics; glycylcyclines; leprostatics; lincomycin derivatives; macrolide derivatives, such as ketolides or macrolides; miscellaneous antibiotics; oxazolidinone antibiotics; penicillins, such as aminopenicillins, antipseudomonal penicillins, beta-lactamase inhibitors, natural penicillins or penicillinase resistant penicillins; quinolones; sulfonamides; tetracyclines; urinary anti-infectives; skin anti-infectives quaternary ammonium compounds (quats) or any combinations thereof.

Advantageously the releasable drug is primarily loaded into the guest polymer.

The drug may e.g. be loaded into the guest polymer by applying the IPN in a fluid comprising the drug under condition were at least a part of the hydrogel guest polymer is swelled. Examples of loading methods are given in WO2013075724, Santos et al. "Poly(hydroxyethyl methacrylate-co-methacrylated-β- cyclodextrin) hydrogels: Synthesis, cytocompatability, mechanical properties and drug loading/release properties" ScienceDirect, Acta Biomaterialia 4 (2008) 745-755 and WO 2005/055972.

Advantageously the releasable drug is releasable via at least one of the luminal surface and the external surface. Where the drug is an anti-proliferative drug, it may advantageously be releasable at least via the luminal surface. The anti-proliferative drug has shown to prevent or reduce migration of vascular smooth muscle cells from the native blood vessel to the luminal surface (the intima - the inner layer abutting the vessel/lumen) and their proliferation and extracellular matrix deposition. The anti-proliferative drug thereby has the function of reducing the risk or preventing intimal hyperplasia.

In an embodiment, the drug is an anti-infection drug and the drug is releasable at least via the external surface. Thereby the risk of infection may be even further reduced or even fully prevented.

The guest polymer (and/or the hydrogel) may comprise a homopolymer or a copolymer. Preferably, the guest polymer is cross-linked.

The guest polymer may be physically cross-linked and/or chemically cross-linked.

Copolymers mean polymers that are obtained by copolymerization of two or more monomer species.

In an embodiment, the copolymer guest polymer is a biopolymer having two monomer species.

The composition of the guest polymer may be homogeny and/or constant in a guest polymer domain or it may vary e.g. by varying the amount of one or more monomers e.g. in a gradient. This may e.g. be provided by varying the relative amounts of monomers during loading of the monomers into the host polymer and/or polymerisation thereof. For example, the guest polymer may in a first part of a domain predominantly consist of PHEMA, in a second middle part of the domain it may comprise PHEMA and PEGMEA and in a third e.g. luminal part it may comprise PHEMA, PEGMEA and sulfobetaine. The skilled person will understand that any combinations of monomers may be possible and may be selected according to the desired properties of the medical tubular device.

In an embodiment, the copolymer guest polymer is a terpolymer obtained from three monomer species.

In an embodiment, the copolymer guest polymer is a quaterpolymer obtained from four monomer species. The guest polymer may be fully hydrophilic or it may comprise hydrophobic domains. In an embodiment, the hydrogel (guest) polymer is a hydrophobic-hydrophilic hybrid hydrogel. The hydrogel (guest) polymer with hydrophobic domains may be produced from a mixture of monomers comprising hydrophilic monomers and hydrophobic monomers. Preferably, the hydrophobic monomers are present in about 20 % by weight or less, such as 10 % or less of the monomers. In an embodiment the hydrogel (guest) polymer comprises moieties which may switch between stages e.g. upon influence by irradiation, pH value and/or mechanical influence, to provide a local domain to have a hydrophilic character and a hydrophobic character respectively. Such moieties may e.g. be a photoactive moiety as described herein. In an embodiment, the hydrogel (guest) polymer comprises a copolymer or multi-polymer comprising spiropyran and a hydrophobic monomer. This may e.g. be advantageous where the guest polymer comprises a drug, such as a hydrophilic drug for controlled and/or triggered release.

The guest polymer may preferably be polymerized from one or more monomers comprising an acrylate; a vinyl monomer, such as n-vinyl pyrolidone (nVP); styrene; oxygen-, phenyl, amino and nitrogen-containing acrylic and methacrylic derivatives, e.g. acrylic esters, acrylic acids, methacrylic acid and -esters, alkyl and hydroxyalkyl acrylates and methacrylates; functionalized (meth)acrylates such as 2-hydroxyethyl methacrylate (HEMA), glycerol monomethacrylate (GMMA), heptaflurobutyl acrylate (HFBA), 2-methacryloyloxyethyl phosphorylcholine (MPC) and [2-(methacryloyloxy)ethyl]-dimethyl-(3-sulfopropyl)-ammonium hydroxide (Betain); alkyl substituted acrylates and methacrylates such as methyl methacrylate (MMA), ethyl methacrylate (EMA), butyl methacrylate (BMA), dodecyl methacrylate (DMA); PEGylated (meth)actylates such as poly(ethylene glycol) methyl ether methacrylate (PEGMEMA) and poly(ethylene glycol) methyl ether acrylate (PEGMEA); substituted β- and γ-lactones, lactic acid monomers; carbohydrides and fluorinated monomers; urethanes; mono- and di-functional alcohols; carboxylic acids; amines; isocyanates; epoxides; aromatics carrying alkyl group(s); sulfonated aromatics, aromatic resins; imidazole; imidazol derivatives; zwitterionic monomers; pyrazoles; quaternary ammonium monomers; spiropyran monomers, Chitin or a derivative thereof (Chitosan) and any/or any combinations comprising one or more of the above mentioned.

A hydrogel (guest) polymer comprising monomer "X" is herein meant to designate a guest polymer obtained from one or more monomers including monomer "X".

In an embodiment the guest polymer comprises poly(2-hydroxyethyl methacrylate) (PHEMA), preferably the guest polymer is a cross-linked copolymer of PHEMA and PEGMEA.

In an embodiment, the hydrogel guest polymer comprises a copolymer polymerised from monomers comprising at least one zwitterion and at least one of PHEMA, PEGMEA or vinylimidazole butane sulfonate, preferably the hydrogel guest polymer comprises a copolymer polymerised from monomers comprising PHEMA, PEGMEA vinylimidazole butane sulfonate and/or a sulfobetaine.

In an embodiment the guest polymer comprises a homo- or co-polymer polymerised from monomers comprising at least one monomer comprising a protecting group protecting a functional moiety, the protecting group is preferably fluorenylmethyloxycarbonyl (FMOC) and/or the protected functional moiety preferably comprises an -NH₂ group.

Examples of suitable protection moieties may be found in US2008/0227092. Examples of suitable functional moieties may be found in US2008/0227092.

After polymerization and optionally crosslinking the protecting group at the luminal and/or external surface may be removed to de-protected the functional moiety and a drug as described above may be bonded to the guest polymer via the functional moiety (e.g. -NH₂) and optionally via a linker (e.g. PEG, PEO).

In an embodiment the guest polymer comprises a copolymer, preferably polymerized from monomers comprising at least one zwitterionic moiety, such as a sulfobetaine, a carbobetaine, a phosphobetaine, or a phosphocholine e.g. as described above.

In an embodiment, the guest polymer comprises a copolymer polymerised from monomers comprising a photoactive monomer comprising a photoactive moiety, such as a spiropyran group. The photoactive monomer is advantageously photochromic.

A "photoactive monomer" means herein a monomer comprising at least one photoactive moiety.

The guest polymer advantageously comprises spiropyran acrylate.

By including photoactive moieties in the guest polymer, the guest polymer becomes itself photoactive and thereby the location and/or one or more properties of the medical tubular device may be determined, altered and/or adjusted by light, e.g. by irradiating the body structure with UV light. Thereby a surgeon (including a doctor or any other trained person or trained robot) may switch on and off a certain biological property (such as hydrophilicity and/or drug release properties).

Further information about photochromic moieties suitable for being applied in monomers forming the guest polymer may be found in "PREPARATION AND ACTIVATION OF SPIROPYRAN-MEROCYANINE SYSTEM" by Nordin et al. Malaysian Journal of Analytical Sciences, Vol. 17No. 3 (2013): 422- 429.

In an embodiment, the guest polymer comprises embedded chitosan, such as a sub guest interpenetrating chitosan network. The chitosan network may be modified with a photo-responsive derivative, such as a spiropyran derivative.

The synthesis of an interpenetrating chitosan network modified with Spiropyran (SP) derivatives and suitable for providing a sub guest interpenetrating network may be as described in "Fabrication of photochromic hydrogels using an interpenetrating chitosan network" by Cheol Woo Lee et al. J. APPL, POLYM. SCI. 2017, DOI: 10.1002/APP.45120. The chitosan may be loaded into the guest and/or host polymer after the guest polymer has been cross-linked.

The guest polymer of the IPN may comprise two or more types of guest polymer domains having equal or different composition.

In an embodiment the two or more types of guest polymer domains comprises a surface guest polymer domain comprising the guest polymer at least at the luminal surface or at the external surface and an intermediate guest polymer domain with a different type of guest polymer.

In an embodiment the two or more types of guest polymer domains comprises a first type of guest polymer comprising the guest polymer at the luminal surface and a second type of guest polymer comprising the guest polymer at the external surface.

In an embodiment the two or more types of guest polymer domains comprises a third intermediate guest polymer, which can be the same or different from that of either the luminal or external surfaces.

The guest polymer(s) of the body structure may advantageously constitute at least about 1 % by dry weight of the IPN, such as at least about 10 % by dry weight of the IPN, such as from about 15 % to about 85 %, such as from about 25% to about 60 % dry weight of the IPN.

Advantageously the at least one guest polymer domain of the body structure includes at least one surface of the body structure such as at least one of the luminal surface and the external surface of the body structure.

In an embodiment, the guest polymer in the at least one guest polymer domain is substantially evenly distributed in the host polymer matrix.

In an embodiment the at least one guest polymer domain has an amount gradient of the guest polymer in a direction perpendicular (normal) to luminal and/or the external surface of the body structure, preferably such that the amount of guest polymer closer to the surface of the body structure is larger than the amount of guest polymer further from the surface of the body structure.

By structuring the amount and/or monomer distribution in the host polymer, the body structure may be designed and optimized for many different applications, e.g. for controlling a release profile of a releasable drug.

In an embodiment, the body structure comprises a single guest polymer domain e.g. including optional coatings of guest polymer. Preferably, the guest polymer extends to at least a part of the luminal surface and/or includes a coating of the luminal surface.

In an embodiment, the guest polymer domain extends in the entire thickness of the host polymer matrix and preferably, the IPN extend in the entire thickness of the body structure.

In certain applications, it is desired that the body structure has a high tissue ingrowth potential to ensure that the medical tubular device is relatively fast growing together (maturing) with the tissue it is adapted to be located in contact with. Thereby potential risk of infection between the tissue and the medical tubular device may be highly reduced. Thereby potential risk of accumulation of blood from potential bleeding creating a hematoma between the external surface of the medical tubular device and the surrounding tissue may be highly reduced.

It has been found that the risk of forming excessive amount of scar tissue may be reduced. It has been found that where the amount of scar tissue formation is high the tissue tends to retract around the body structure and thereby reducing the internal diameter of the body structure. By ensuring that the body structure has a high ingrowth potential both risk of infection and risk of retraction may be reduced.

The inventor has found that the body structure may be ensured to have a high ingrowth potential by providing that the at least one guest polymer domain comprises a clot promoting domain and/or a cell proliferation promoting domain, where the clot promoting domain and/or cell proliferation promoting domain is located at a distance from the luminal surface and does preferably not comprise the luminal surface. Thereby the risk of cell growth inside the lumen of the medical tubular device may be low while simultaneously the body structure may be ensured to have a high ingrowth potential.

The body structure may for example have an external surface guest polymer domain comprising the clot promoting and/or cell proliferation domain and a luminal surface guest polymer domain comprising an anti-proliferative drug.

It has been found that the medical tubular device comprising a guest polymer domain with a clot promoting and/or cell proliferation domain may also ensure that risk of excessive bleeding, e.g. during implanting the medical tubular device, may be highly reduced. This effect has been observed, even where the medical tubular device comprises anti-proliferative drug and/or thrombosis deactivating drug at the luminal surface and/or in a luminal surface guest polymer domain. Advantageously the clot promoting guest polymer comprises an external surface guest polymer domain comprising at least a part of the external surface.

In an embodiment, the clot promoting and/or cell proliferation domain is located at an intermediate guest polymer domain, which does not comprise the luminal surface or the external surface. The medical tubular device of this embodiment may or may not have a high ingrowth potential, however the intermediate guest polymer domain with the cell proliferation promoting drug and/or a clot promoting drug may ensure that risk of excessive bleeding e.g. during implanting the medical tubular device and/ or post-implanting access (dialysis), may be highly reduced. Thus, the medical tubular device of this embodiment may advantageously have an external surface guest polymer domain comprising an anti-proliferative drug for reducing the ingrowth potential.

In an embodiment, it is desired that the clot promoting domain comprising releasable clot promoting drug is located at respective ends of the medical tubular device where it is adapted to be sutured to another graft or to a native blood vessel thereby ensuring that the risk of excessive bleeding, e.g. during implanting the medical tubular device, may be highly reduced.

In an embodiment, it is desired that the cell proliferation domain comprising releasable cell proliferation drug is an external domain along substantially the entire length of the body structure thereby ensuring fast ingrowth of the external surface to the surrounding tissue with low risk of scar tissue.

The clot promoting and/or cell proliferation domain may for example comprise a cell proliferation promoting drug and/or a clot promoting drug, such as a fibrinolytic drug, a heparin antagonist, a platelet-stimulating drug or any combination thereof comprising one or more of the above mentioned clot promoting drugs.

For many applications, it is desired that the medical tubular device has a high ingrowth potential for ensuring fast ingrowth to the surrounding tissue after implantation to thereby ensure a reduced tendency and/or risk of lateral bleeding around the implanted device. This is in particular preferred where the medical tubular device is a dialysis graft.

In an embodiment, the host polymer comprises two or more guest polymer domains.

In an embodiment the two or more guest polymer domains comprises a luminal guest polymer domain and an external guest polymer domain. The two or more guest polymer domains may advantageously be separated by a polymer portion of the body structure essentially free of guest polymer, the polymer portion is preferably identical to the host polymer matrix, but essentially without guest polymer.

The body structure may be of a single layer or it may comprise several layers, such as several tubular layers. Advantageously at least the host polymer forms a tubular layer in the entire length of the body structure. The additional layer(s) may be fully tubular in the whole body structure length or it/they may be only local layers e.g. in a part of the length of the body structure.

In an embodiment, the body structure is a layered body structure comprising two or more layers, wherein at least one of the layers comprises at least one IPN portion comprising or consisting of the IPN.

In an embodiment the body structure comprises a luminal layer including the luminal surface, wherein the luminal layer comprises or consist of the at least one IPN. Preferably the luminal surface comprises the guest polymer e.g. as described above. Advantageously, the guest polymer domain comprises a luminal surface guest polymer domain comprising at least a part of the luminal surface, such as the entire luminal surface.

In an embodiment the body structure comprises an external layer including the external surface, wherein the external layer comprises the at least one IPN. Preferably the external surface comprises the guest polymer e.g. as described above. Advantageously, the guest polymer domain comprises an external surface guest polymer domain comprising at least a part of the external surface, such as the entire external surface.

The body structure may comprise one or more intermediate layer(s) and/or domain(s) not including a surface of the body structure. Advantageously, the guest polymer domain comprises an intermediate guest polymer domain, which does not comprise the luminal surface or the external surface.

In an embodiment, at least one layer of the body structure is a polymer layer without an IPN, such as a polymer layer of polytetrafluoroethylene (PTFE), expanded PTFE (e.g. Goretex ^{®}), polyurethane (PU), Polyethylene terephthalate (PET, e.g. dacron) and/or silicone elastomer.

Such materials and/or layer(s) of material(s) are described in for example US 20160354217, US 20140142682, US 20090258958, US 6,517,571, US 0060118236 and/or US 5,931,865.

In an embodiment the at least one polymer layer of the body structure without an IPN is an elastomeric layer.

Advantageously, the two or more layers are located concentrically to form the layered body structure, the concentrically located layers are preferably in full surface-to-surface (interfacial) contact with adjacent layer(s). The two or more layers may have same or different lengths.

The two or more layers may be non-bonded layers, locally fixed layers or interfacial bonded layers.

In an embodiment, at least two layers of the two or more layers are not chemically fixed to each other. The least two non-fixed layers may be mechanically held together, e.g. via mechanical interlocking.

In an embodiment at least two layers of the two or more layers are interfacial bonded to each other e.g. by an intermediate adhesive.

In an embodiment, at least two layers of the two or more layers are anchored to each other. The anchoring of the at least two layers preferably comprises a local adhesive bonding, sutures, staples and/or clips, pinholes.

The medical tubular device may in addition comprises a tubular support structure located concentrically with the body structure, e.g. inside or surrounding the body structure.

The support structure may for example comprise a support structure located in contact with the luminal surface or in contact with the external surface of the body structure.

The support structure advantageously has a higher stiffness than the body structure. Thereby the support structure may support the medical tubular device at desired location(s). Advantageously, the tubular support structure or merely "support structure" does not cover the entire of any of the external surface or the luminal surface of the body structure. Preferably the support structure cover up to 50 % of the external surface or the luminal surface of the body structure, such as up to 40 %, such as up to 30%, such as up to 20%, such as up to 10 % of the external surface or the luminal surface of the body structure.

The support structure may for example comprise a helical wire. The helical wire may e.g. be located in contact with the luminal surface to ensure that the body structure is not collapsing. The helical wire may e.g. be located to surround and be in contact with the external surface. Thereby the helical wire may provide an increased proliferation and faster ingrowth of the body structure. The helical wire may e.g. be embedded inside the body structure. Thereby the helical wire may provide mechanical support without interfering with biological properties.

In an embodiment, the tubular support structure comprises a cuff close-fitting to the external surface of the body structure.

The cuff may have an open crosshatched structure, and preferably covers up to about 25 % of the external surface of the body structure, such as from about 1 % to about 20 % of the external surface of the body structure.

The cuff may for example be of a polymer material, preferably comprising collagen, Dacron, nylon, polytetrafluoroethylene (PTFE), expanded PTFE (e.g. Goretex ^{®}), Polyethylene terephthalate (PET, e.g. dacron), polyurethane (PU) and/or silicone elastomer. In an embodiment, the cuff comprises fibres e.g. for faster ingrowth. In an embodiment, the cuff comprises Fluorinated ethylene propylene (FEP). FEP is a copolymer of hexafluoropropylene and tetrafluoroethylene and is e.g. sold under the brand names Teflon (DuPont), FEP, Neoflon FEP (Daikin) or Dyneon FEP (Dyneon/3M). The cuff may advantageously comprise an IPN material loaded with one or more drugs, such as growth hormone(s), antifibrinolytic drug(s), clot promoting drug(s) and/or cell proliferation drug.

Advantageously, the cuff forms a scaffold for infiltration, proliferation, and growth of cells.

In an embodiment, at least a part of the external surface of the body structure and/or at least a part of an external surface of the support structure comprises an ingrowth enhancement drug, such as growth hormone, antifibrinolytic drug(s) clot promoting drug(s) and/or cell proliferation promoting drug(s).

The ingrowth enhancement drug may advantageously form a scaffold for infiltration, proliferation, and/or growth of cells. The medical tubular device comprising ingrowth enhancement drug is preferably an implantable medical tubular device, such as a dialysis graft, a vascular graft or a carrier and/or an insulation of an artificial neural implant.

The ingrowth enhancement drug of the external surface of the body structure and/or the external surface of the support structure ensure(s) an even faster ingrowth and external maturation of the medical tubular device, thereby ensuring a reduced tendency and/or risk of lateral bleeding.

The very high ingrowth potential of the medical tubular device having the ingrowth enhancement drug of the external surface of the body structure and/or the external surface of the support structure ensure that the medical tubular device is relatively fast growing together with the tissue it is adapted to be located in contact with. Thereby the potential risk of infection between the tissue and the medical tubular device may be highly reduced and in addition, the risk of forming excessive amount of scar tissue may be reduced thereby reducing the risk of retraction of tissue surrounding the body structure, which again adds to prolong the time of patency. Furthermore, if the medical tubular device is a dialysis graft, the dialysis treatment may start at an earlier point of time compared to using a prior art dialysis graft, due to faster maturation. If the graft is not fully matured and the self-healing is not sufficient, there may be some accumulation of blood in the void between the external surface and the tissue caused by leakage of blood through the needle penetration channels.

The clot promoting drug may for example be as described above e.g. a fibrinolytic drug, a heparin antagonist, a platelet-stimulating drug or any combination thereof comprising one or more of the above-mentioned clot promoting drugs.

Advantageously, the ingrowth enhancement drug comprises extracellular matrix (ECM) molecules, preferably comprising fibrous protein(s), and/or proteoglycan(s). In an embodiment, the ingrowth enhancement drug comprises collagen.

The main fibrous proteins that build the extracellular matrix are collagens, elastins and laminins. These are all relatively sturdy protein macromolecules. Their sturdiness may provide the extracellular matrix with buffering and force-resisting properties.

In an embodiment, the ingrowth enhancement drug comprises a natural extracellular matrix or a synthetic extracellular matrix or any combinations or fractions thereof.

The ECM may for example be an OASIS^{®} Wound Matrix, which is an intact matrix naturally derived from porcine small intestinal submucosa (SIS), indicated for the management of wounds. The OASIS^{®} Wound Matrix is marketed by Smith & Nephew, Inc.

Another example is the ECM obtainable from the Swedish company VERIGRAFT. VERIGRAFT generates personalized tissue-engineered transplants for use in regenerative medicine.

A further example of ECM material is the Corning Matrigel matrix, which is, a solubilized basement membrane preparation extracted from the Engelbreth-Holm-Swarm (EHS) mouse sarcoma, a tumor rich in such ECM proteins as laminin (a major component), collagen IV, heparin sulfate proteoglycans, entactin/nidogen, and a number of growth factors.

Further information and examples of ECM may for example be found in the article by Eduardo A.Silva and David J.Mooney. "Synthetic Extracellular Matrices for Tissue Engineering and Regeneration". ELSEVIER, Volume 64, 2004, Pages 181-205, https://doi.org/10.1016/S0070-2153(04)64008-7.

To further increase the ingrowth properties of the medical tubular device at least a part of the external surface of the body structure and/or at least a part of an external surface of the support structure may comprises pre-grown cells.

Such pre-grown cell may e.g. be provided by seeding at least a part of the external surface of the body structure and/or at least a part of the external surface of the support structure with stem-cells and subsequently embed the body structure with optional support structure in a differentiation and growth medium. To ensure that the cells are not migrating into the lumen (channel) of the medical tubular device the lumen of the medical tubular device may advantageously be closed and/or sealed at the ends of the medical tubular device.

In an embodiment, at least a part of the external surface of the body structure and/or at least a part of an external surface of the support structure comprises a bioadhesive, such as a hydrogel or hydrogellable bioadhesive, preferably, the bioadhesive comprises chitosan.

The bioadhesive of the external surface of the body structure and/or of the external surface of the support structure ensures a reduced tendency and/or risk of lateral bleeding around the implanted device immediately after implant and even before ingrowth has started. Thereby the bioadhesive ensures an even faster ingrowth and maturation which in particular is desired where the medical tubular device is a dialysis graft. In addition, it has been found, that the bioadhesive may also result in a reduced formation of scar tissue. It is believed, that this may be caused by ensuring that friction and relative movements between the body structure and the tissue surrounding the body structure may be reduced due to the bioadhesive. Moreover the bioadhesive also ensures an even further reduced risk of infection and inflammation.

In an embodiment, the body structure comprises an external surface guest polymer domain, comprising at least a part of the external surface and wherein at least a part of the bioadhesive comprises at least a part of the guest polymer of the external surface. For example, the guest polymer of the external surface may comprise chitosan, bonded to the guest polymer and/or embedded (interpenetrating) in the guest polymer.

The bioadhesive may e.g. be adhered to the tubular support structure and/or the body structure, e.g. using a silicone elastomer, such as using Sylgard 184 (Dow Corning, Diatom), Silastic (Dow Corning) and/or Adhesive silicone type A MED-1137 (Nusil). The bioadhesive may e.g. be chitosan e.g. as marketed by KitoZyme. Advantageously the chitosan is in form of powder or dry foams e.g. obtained by freeze-drying. The chitosan may advantageously be fixed to the body structure and optional support structure using silicone elastomer or any other suitable attachment elastomer/adhesive.

In an embodiment, at least a part of the bioadhesive is bonded to the tubular support structure and/or the body structure, e.g. by grafting.

Due to the chemical composition of the medical tubular device, it has been found to be possible to provide medical tubular devices with a very narrow lumen, while still ensuring a long patency and reduced risk of restenosis/blocking the lumen (e.g. by neointimal hyperplasia, blood clotting and biofilm formation from bacterial infections). Further desired mechanical properties may also be obtained. The medical tubular device may for example have a narrow internal diameter e.g. down to 1 mm or even narrower depending on the use of the tubular device. In an embodiment, the medical tubular device has an internal diameter of from about 2 mm to about 6 cm, such as from about 3 mm to about 5 cm, such as from about 4mm to about 3 cm, wherein the diameter may be equal or different along the length of the body structure.

In addition, the wall thickness of the body structure may be very thin while still having desired mechanical properties, such as strength and compliance. In an embodiment, the body structure has a wall thickness of from about 0.01 mm to about 5 mm, such as from about 0.1 mm to about 3 mm, such as from about 0.5 mm to about 1 mm.

It has been found that the compliance should not be too low but also not too high since a too high compliance may result in a high deformation and tearing in the sutures and hence may cause undesired bleeding at or near the sutures. Advantageous the body structure should have a compliance (Percentage inner diameter change per mmHg x 10⁻² in the range 80-200 mmHg (determined according to ISO 7198: 2016, section A5.9) of at least 1 and no more than 10. Generally natural blood vessels has a compliance larger than 2, such as in the interval from about 2.5 to 6.

Hence, it is desired that the body structure has a compliance from about 2 to about 8, such as from about 2.5 to about 6, such as from about 2.5 to about 6, such as from about 3 to about 5, such as from about 3.3 to about 4.5.

Further, it is desired that the body structure has a relatively low long-term dilatation. The dilatation may e.g. be determined as described in "Long-term dilatation of polyester and expanded polytetrafluoroethylene tube grafts after open repair of infrarenal abdominal aortic aneurysms" J Vasc Surg 2011;53:1506-13.

In an embodiment wherein the body structure has a long-term dilatation after 12 months of less than about 25 %, such as less than about 20 %, such as less than about 10 %, such as less than about 5 %.

In an embodiment wherein the body structure has a long-term dilatation after 6 years of less than about 50 %, such as less than about 30 %, such as less than about 25 %, such as less than about 15 %.

The tubular device may advantageously be a vascular graft, a dialyse graft, a dialysis tube, a catheter, a shunt, a stent, a stentgraft and/or an intestine graft.

Preferably, the medical tubular device is an implantable tubular device, such as a vascular graft, dialysis graft or a stent.

In an embodiment the medical tubular device is a dialyse tube.

In an embodiment, the medical tubular device is a feeding tube, such as a gastrostomy tube.

In an embodiment, the medical tubular device is a venous catheter, such as a central venous catheter (CVC line) or a Peripherally Inserted Central venous Catheter (PICC line).

In an embodiment, the medical tubular device is a drug supply tube, such as a tube for supplying anti-cancer drug.

The invention also comprises a method of producing the medical tubular device described above. The method comprises producing the body structure by a method comprising:
- shaping a tubular host polymer substrate with a luminal substrate surface and an external substrate surface,
- loading monomers of the guest polymer into at least a portion of the tubular host polymer substrate by at least partly swelling the host polymer substrate by a solvent comprising the monomers and
- Polymerizing and optional cross-linking the monomers to form the IPN comprising the at least one guest polymer domain.

The host polymer substrate may directly form the host polymer matrix or optionally residuals are extracted from the host polymer substrate prior to depositing (guest) monomers within the host polymer matrix.

Extraction of residuals of the host polymer substrate, loading with monomers for the guest polymer, polymerizing and optionally crosslinking as well as other optional and/or desired process step may be found in the following publications:
Antimicrob. Agents Chemother 2017, 61(7), e00604-17 DOI:10.1128/AAC.00604-17. Plasmid 2016, 87-88, 72, DOI: 10.1016/j.plasmid.2016.10.001. J. Control. Release 2016, 241, 125-134 DOI: 10.1016/j.jconrel.2016.09.018. J. Mat. Chem. Phys. 2016, 181, 495-500 DOI: 10.1016/j.matchemphys.2016.06.086. J. Indus. Eng. Chem. 2016, 33, 142-149 DOI: 10.1016/j.jiec.2015.09.026. Biomacromolecules. 2016, 17(4), 1321-1329 DOI: 10.1021/acs.biomac.5b01722. J. Biomed. Mater. Res. Part B Appl. Biomater. 2016, 104(2), 402-410 DOI: 10.1002/jbm.b.33371. Eur. J. Pharm. Biopharm. 2015, 94, 305-311 DOI: 10.1016/j.ejpb.2015.05.014. Martin Alm, Peter Thomsen. A delivery device. Patent, PA 2015/70520. Martin Alm, Søren Langer Steffensen. A method of producing a delivery device. Patent, WO 2013/075724. Martin Alm, Maike Benter, Anne Marie Jensen. A method of producing an article comprising an interpenetrating polymer network (IPN) and an article comprising an IPN. Patent, WO 2008/052568. Martin Alm, Maike Benter, Anne Marie Jensen. A method of producing an article comprising an interpenetrating polymer network (IPN) and an article comprising an IPN. Patent, WO 2008/052563. Maike Benter, Martin Alm. A method of coating a polymer surface with a polymer containing coating and an item comprising a polymer coated polymer. Patent, WO 2006/074666. Joachim Karthauser, Maike Benter, and Martin Alm. A method of producing a silicone elastomer rubber item and the product obtainable by the method. Patent, WO 2006/045320.

Advantageously the shaping of the tubular host polymer substrate is performed by extrusion or injection moulding and preferably to provide that the shaped tubular host polymer is seamless. The tubular host polymer substrate may be extruded with any desired wall thickness, inner/outer diameters and in any desired length. In an embodiment the tubular host polymer substrate is shaped by a method comprising knitting or wowing.

In an embodiment the tubular host polymer substrate is produced by using a mandrel and preferably to provide that the shaped tubular host polymer is seamless. The tubular host polymer substrate may be produced with a mandrel with any desired design, shape, wall thickness, inner/outer diameters and in any desired length.

In an embodiment, a surface part of the tubular host polymer substrate may be masked during at least a part of the monomer loading step. The masked surface part preferably being a luminal surface part and/or an external surface part. Optionally the method further comprising de-masking the before masked surface part, masking the before non-masked part and repeating the step of monomer loading and polymerizing. Preferably, the repeated loading step comprising loading with monomers comprising at least one different monomer than the monomers loaded in the first loading step. Thereby the IPN may have a luminal surface guest polymer domain and an external surface guest polymer domain preferably having different guest polymer compositions. E.g., the luminal surface may comprise a masked functional moiety for being de-masked and a drug bonded thereto e.g. a thrombosis deactivating drug and the external surface may comprise zwitterionic moieties. Other combinations and variations will be clear to the skilled person from the description.

In an embodiment the step of loading the monomers comprises loading the tubular host polymer substrate with a varying concentration and/or with varying types of monomers and/or with a varying loading time, to thereby provide the at least one guest polymer domain with an amount and/or monomer gradient of the guest polymer in a direction perpendicular (normal) to the surface of the body structure.

In an embodiment the method comprises applying a guest polymer coating onto at least a part of the luminal surface and/or the external surface, the coating application step preferably comprises performing the polymerizing step in a reactor while the part of the luminal surface and/or external surface are subjected to the monomers.

The guest polymer coating may be of the same or a different guest polymer than the guest polymer of the loaded and polymerized and optionally cross-linked guest polymer e.g. as described above.

The monomers for the guest polymer loaded into the host polymer and/or of the guest polymer coating may be as described above and advantageously comprise a zwitterionic monomer, a combination of cationic and anionic monomers, a spiropyran monomer and/or a monomer with a protected functional moiety (e.g. a reactive moiety/anchor), preferably the monomers comprises at least one of sulfobetaines, carbobetaines, phosphobetaines, phosphocholines or any combinations thereof.

In an embodiment wherein the monomers for the guest polymer loaded into the host polymer and/or of the guest polymer coating comprises at least one monomer comprising a protecting group protecting a functional moiety, the method preferably further comprises a step of binding a drug to the guest polymer exposed at the luminal and/or the external surface of the body structure after having polymerized and optionally cross-linked the monomers.

The step of binding a drug to the guest polymer exposed at the luminal and/or the external surface comprises deprotecting the functional moiety and reacting the deprotected functional moiety with a drug optionally via a linker to provide a covalent bond to the drug. The protecting group is preferably fluorenylmethyloxycarbonyl (FMOC) and/or the protected functional moiety preferably comprises a -NH₂ group.

The method may further comprises loading the at least one guest polymer domain with a releasable drug e.g. as described above.

For example, the drug may be loaded to an external surface guest polymer domain comprising at least a part of the external surface by closing the lumen of the body structure and immersing the body structure in a drug containing fluid e.g. at elevated pressure during loading.

A luminal surface guest polymer domain comprising at least a part of the luminal surface may be loaded by drug e.g. by flushing the lumen with a fluid comprising the drug e.g. at elevated pressure.

An intermediate guest polymer domain, which does not comprise the luminal surface or the external surface, may be loaded by forming the body structure of a layered product wherein a guest polymer domain has been loaded prior to the lamination of the two or more layers.

In an embodiment the method comprises providing two or more guest polymer domains separated by a polymer portion of the tubular host polymer essentially free of guest polymer. In an embodiment the method comprises providing at least one further layer concentrically and preferably in full surface-to-surface contact onto or into the tubular host polymer substrate.

The at least one further layer may comprise a fluid impermeable (barrier) layer portion, a porous layer portion, and/or a portion with micro holes, wherein the portion may be a part of the layer or the full layer.

Preferably, at least one of the further layer(s) is a polymer layer without an IPN, e.g. an elastomer layer, such as a polymer layer of polytetrafluoroethylene (PTFE), expanded PTFE (e.g. Goretex ^{®}), polyethylene terephthalate (PET, e.g. dacron), polyurethane (PU) and/or silicone elastomer. Further examples of polymer layers are provided elsewhere in the description.

The method may further comprise providing a tubular support structure in contact with the luminal surface or in contact with the external surface of the body structure. In an embodiment, the tubular support structure may be embedded in the body structure.

The tubular support structure comprises a cuff close-fitting to the external surface of the body structure. The cuff(s) is/are advantageously fixed to the body structure to ensure that there is not formed a passage between the body structure and the cuff(s). The support structure may be as described above.

In an embodiment, the method comprises applying an ingrowth enhancement drug, such as the ingrowth enhancement drugs disclosed above, onto at least a part of the external surface of the body structure and/or at least a part of an external surface of the support structure.

In an embodiment, the method comprises pre-growing cells onto at least a part of the external surface of the body structure and/or at least a part of an external surface of the support structure. The pre-growing of cells may be provided as disclosed above

In an embodiment the method comprises providing at least a part of the external surface of the body structure and/or at least a part of an external surface of the support structure with a bioadhesive, e.g. as disclosed above.

The invention also comprises a medical device comprising a surface portion comprising a surface coating polymer comprising a zwitterionic moiety, a combination of cationic and anionic moieties, chitin or a derivative thereof (Chitosan), a spiropyran moiety and/or a protected functional moiety, preferably the polymers comprises at least one of the monomers one of sulfobetaines, carbobetaines, phosphobetaines, phosphocholines or any combinations thereof.

In an embodiment, the surface coating of polymer comprises or is a surface coating of hydrogel. The hydrogel may be as described elsewhere herein, for example it may be obtained from monomers as described above for the guest polymer.

The surface coating polymer comprising a zwitterionic monomer, a combination of cationic and anionic monomers, collagen, chitin or a derivative thereof (Chitosan), a spiropyran monomer and/or a monomer with a protected functional moiety may be as the corresponding surface coating polymer as described above.

It should be noted that the medical device of this aspect of the invention may be tubular or alternatively it may have another shape than tubular. The medical device may advantageously be an implant, such as a heart valve, an eardrum, a cornea, a vascular patch, an intestine, a meniscus, a cartilage, a spinal disc or any part(s) thereof.

In an embodiment, the medical device is as the medical tubular device described above.

In an embodiment the polymer coating comprises at least one monomer comprising a protecting group protecting a functional moiety, the protecting group is preferably fluorenylmethyloxycarbonyl (FMOC) and/or the protected functional moiety preferably comprises an -NH₂ group, e.g. as disclosed above.

In an embodiment the polymer coating comprises at least one covalent bonded drug, such as an enzyme, an immunosuppressive drug, a deactivating drug, a cell proliferation promoting drug and/or a clot promoting drug and/or any combination comprising at least one of the mentioned drugs. The drugs may be e.g. as disclosed above.

In an embodiment, the covalently bonded drug is a thrombosis deactivating drug(s), the thrombosis deactivating drug(s) preferably comprises heparin, EDTA, citrate and/or any combination comprising one of the mentioned thrombosis deactivating drugs. The drugs may be e.g. as disclosed above.

In an embodiment the covalently bonded drug is covalently bonded to the polymer coating via a linker molecule, e.g. as disclosed above.

In an embodiment the polymer coating comprises a copolymer polymerised from monomers comprising a photoactive moiety, such as a spiropyran monomer, e.g. as disclosed above.

In an embodiment the polymer coating comprises (sub) guest polymer interpenetrating chitosan polymer, e.g. as disclosed above.

In an embodiment, the medical device has a total surface area comprising the surface portion and the surface portion comprising the polymer coating surface is from 5 to about 100 % of the total surface area.

In an embodiment, the medical device is partly or fully of polymer and the polymer coating is located at a surface portion of the medical device, the polymer portion preferably being flexible. The term "flexible" is used in opposite to rigid. Hence, the polymer portion may be bend or be forced out of shape without being permanently damaged.

The invention also comprises a medical device comprising an external surface portion comprising a bioadhesive, such as a bioadhesive comprising chitosan.

The medical device is a tubular device.

All features of the inventions and embodiments of the invention as described herein including ranges and preferred ranges may be combined in various ways within the scope of the invention, unless there are specific reasons not to combine such features.

The invention also comprises a method of producing a zwitterionic polymer (not according to the invention as claimed). The inventors have found that zwitterionic polymers comprising zwitterionic moieties originating from sulfobetanies, carbobetaines, phosphobetaines or phosphocholines have very good anti-fouling properties. Specifically it has been found that the zwitterionic polymer has protein repelling properties. The zwitterionic polymer may advantageously be used as an anti-fouling coating and/or a protein repelling coating. In an embodiment the zwitterionic polymer is used as guest polymer as described above.

The method of producing a zwitterionic polymer, the method comprises providing at least one zwitterionic monomer selected from sulfobetanies, carbobetaines, phosphobetaines or phosphocholines and polymerising the at least one monomer, preferably the method comprises cross-linking the polymerized monomer to form a hydrogel.

Advantageously, the method comprises providing two or more monomers and polymerising the monomers, wherein the monomers comprises at least the at least one monomer. Preferably the method comprises cross-linking the polymerized monomers.

It has been found to be highly beneficial to use one or more of the novel zwitterionic monomer with formula I - XIII

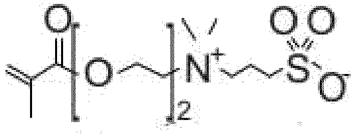

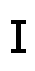

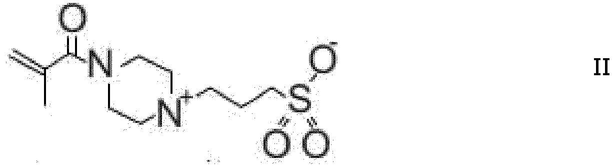

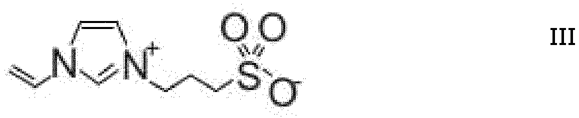

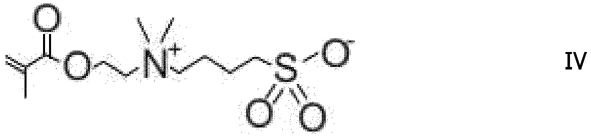

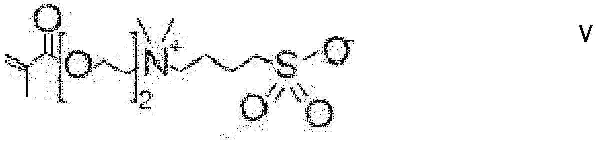

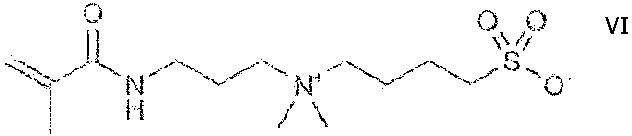

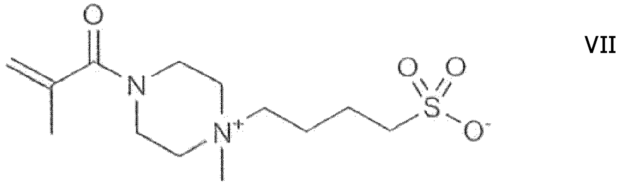

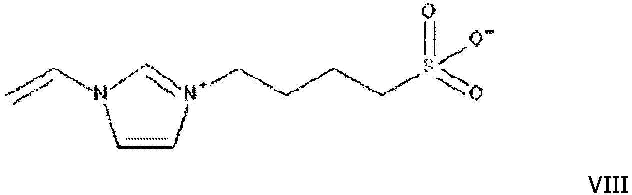

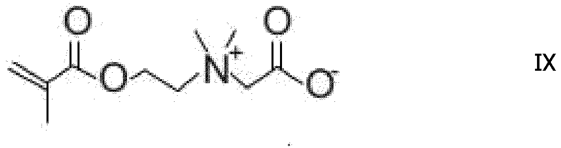

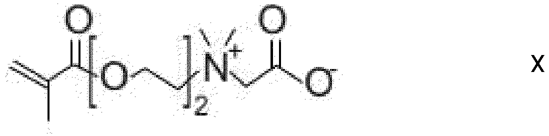

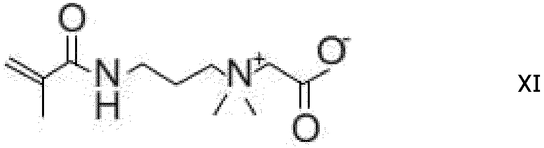

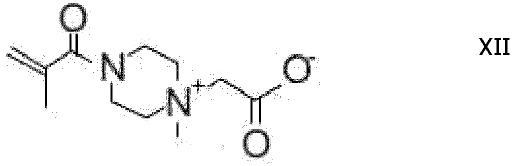

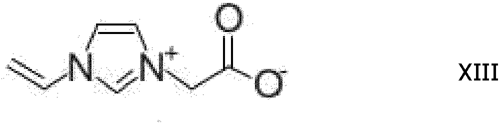

In a preferred embodiment the zwitterionic monomer is the zwitterionic monomer with the formula 1-Ethenyl-3-(4-sulfonatobutyl)-1*H*-imidazol-3-ium This is the zwitterionic monomer referred to on figure 6 as CK 1573.

In a preferred embodiment the zwitterionic monomer is or includes one or more of the zwitterionic monomers:
1-Ethenyl-3-(3-sulfonatopropyl)-1*H*-imidazol-3-ium (CK 1572): 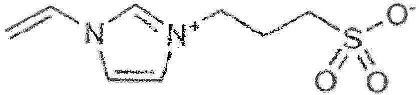
1-methyl-4-(2-methylprop-2-enoyl)-1-(4-sulfonatobutyl)piperazin-1-ium (CK1578): 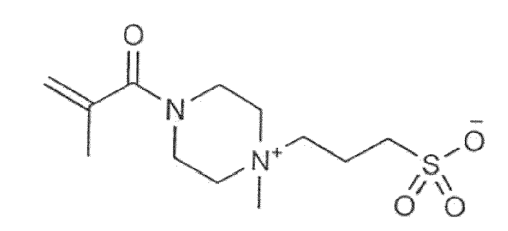
3-[dimethyl(2-{2-[(2-methylprop-2-enoyl)oxy]ethoxy}ethyl)azaniumyl]propane-1-sulfonate (CK1582): 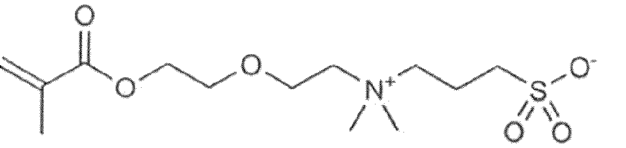
or 4-[dimethyl({2-[(2-methylprop-2-enoyl)oxy]ethyl})azaniumyl]butane-1-sulfonate (CK 1583):

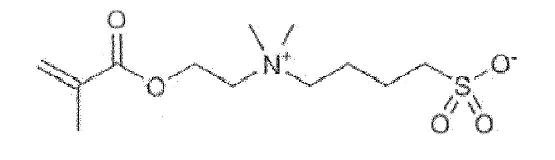

In a preferred embodiment the zwitterionic monomer is or includes one or more of the zwitterionic monomers:
4-(2-methylprop-2-enoyloxymethoxymethylammonio)butane-1-sulfonate (CK1591): 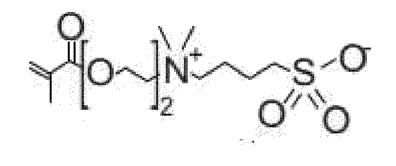
4-{dimethyl[3-(2-methylprop-2-enamido)propyl]azaniumyl} butane-1-sulfonate (CK 1584): 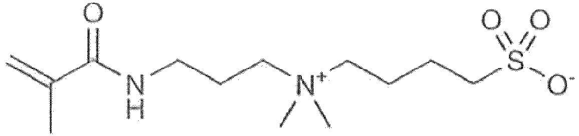
1-methyl-4-(2-methylprop-2-enoyl)-1-(4-sulfonatobutyl)piperazin-1-ium (CK 1586): 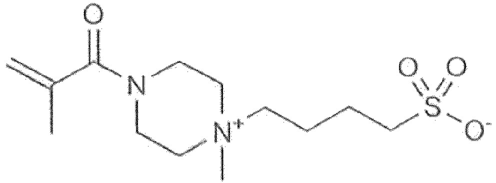
1-carboxy-N,N-dimethyl-N-(2'-methacryloyloxyethyl)methanaminium (CK 1581): 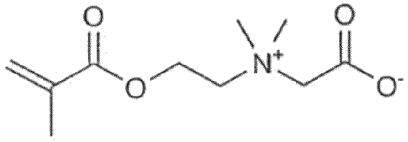
2-[dimethyl(2-{2-[(2-methylprop-2-enoyl)oxy]ethoxy}-ethyl)azaniumyl]acetate (CK 1589): 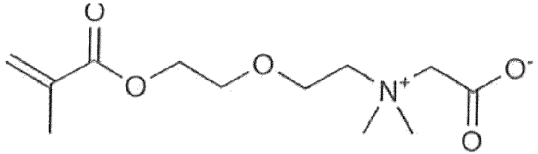
2-{dimethyl[3-(2-mehtylprop-2-enamido)propryl]-azamniumyl}acetate (CK 1588): 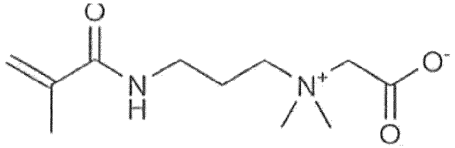
1-(carboxylatomethyl)-1-methyl-4-(2-methylprop-2-enoyl)piperazin-1-ium (CK 1585): 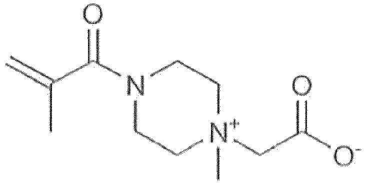
or 3-(carboxylatomethyl)-1-ethenyl-1*H*-imidazol-3-ium (CK 1587):

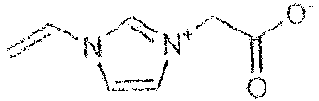

In an embodiment, the zwitterionic polymer is a homopolymer of zwitterionic monomer.

In an embodiment, the zwitterionic polymer is a co-polymer of two or more of the zwitterionic monomers.

In an embodiment, the zwitterionic polymer is a co-polymer of at least one zwitterionic monomer and at least one non-zwitterionic monomer, such as PHEMA, HEMA and/or PEGMEA.

Advantageously the monomers comprises at least about 0.1 % by mol of the monomers of the at least one zwitterionic monomer, such as at least about 0.5 %, such as at least about 1 %, such as at least about 2 %, such as at least about 3 %, such as at least about %, such as at least about 4 %, such as at least about 5 %, such as 2-20 % by mol of the monomers of the at least one zwitterionic monomer.

It has been found that the polymer has especially high anti-fouling properties where the zwitterionic polymer is a copolymer of monomers comprising 1.5 - 10 mol % of the zwitterionic monomer(s). Thereby ensuring a certain distance between the zwitterionic moieties which ensures a highly anti-fouling zwitterionic polymer.

The zwitterionic polymer may comprise one or more acrylates; vinyl monomers, such as n-vinyl pyrolidone (nVP); styrenes; oxygen-, phenyl, amino and nitrogen-containing acrylic and methacrylic derivatives, e.g. acrylic esters, acrylic acids, methacrylic acid and -esters, alkyl and hydroxyalkyl acrylates and methacrylates; functionalized methacrylates such as 2-hydroxyethyl methacrylate (HEMA), glycerol monomethacrylate (GMMA), heptaflurobutyl acrylate (HFBA), 2-methacryloyloxyethyl phosphorylcholine (MPC) and [2-(methacryloyloxy)ethyl]-dimethyl-(3-sulfopropyl)-ammonium hydroxide (Betain); alkyl substituted acrylates and methacrylates such as methyl methacrylate (MMA), ethyl methacrylate (EMA), butyl methacrylate (BMA), dodecyl methacrylate (DMA); PEGylated (meth)actylates such as poly(ethylene glycol) methyl ether methacrylate (PEGMEMA) and poly(ethylene glycol) methyl ether acrylate (PEGMEA); substituted β- and γ-lactones, lactic acid monomers; carbohydrides and fluorinated monomers; urethanes; mono- and di-functional alcohols; carboxylic acids; amines; isocyanates; epoxides; aromatics carrying alkyl group(s); sulfonated aromatics, aromatic resins; imidazole; imidazol derivatives; zwitterionic monomers; pyrazoles; quaternary ammonium monomers; spiropyran monomers; collagen, fibrin, chitin or a derivative thereof (chitosan) and any/or any combination comprising one or more of the above mentioned.

Advantageously the monomers comprises poly(2-hydroxyethyl methacryate) (PHEMA), HEMA and/or PEGMEA.

In an embodiment, the monomers comprises the at least one zwitterionic monomer and at least one of PHEMA, PEGMEA or vinylimidazole butyl sulfonate.

The invention also comprises a zwitterionic polymer obtainable according to the method described above.

In an embodiment, the zwitterionic polymer comprising a copolymer polymerized from at least one zwitterionic monomer and at least one of HEMA, PEGMEA or vinylimidazole butyl sulfonate.

The zwitterionic polymer is advantageously a zwitterionic hydrogel.

It has been found that the zwitterionic hydrogel is very useful as moisture agent, specifically for products, such as cosmetic products for use in contact with skin, hair and/or mucosa (not according to the invention).

Hence, a moisture agent comprising a zwitterionic hydrogel as described above as well as a cosmetic product comprising such moisture agent is disclosed.

A "cosmetic product" shall mean any substance or preparation intended to be placed in contact with the various external parts of the human body (epidermis, hair, nails, lips and external genital organs) or with the teeth and the mucous membranes, such as of the oral cavity.

The moisture agent is especially advantageous for use in cosmetic products selected from crème and shampoo.

In an embodiment, the cosmetic product is a crème comprising from about 0.1 to about 10 % by weight of the moisture agent.

Also disclosed, but not according to the invention, is a protein repellent device with a protein repellent surface area, wherein the surface area is a surface comprising a zwitterionic polymer as described above.

Advantageously the protein repellent device comprises a coating of the zwitterionic polymer (not according to the invention).

The protein repellent device may advantageously be a contact lens comprising or consisting of the zwitterionic polymer not according to the invention).

In an embodiment, the protein repellent device is a reactor, a container or a tube and the protein repellent surface area preferably is a surface area adapted to be in contact with a protein containing fluid when in use. It should be understood that the protein repellent device may be any devise comprising a surface area formed of the zwitterionic polymer not according to the invention).

Also disclosed, but not according to the invention as claimed, is an anti-fouling device with an anti-fouling surface area, wherein the surface area is a surface of a zwitterionic as described above.

In an embodiment, the anti-fouling device comprises a coating of the zwitterionic polymer (not according to the invention).

The zwitterionic polymer may be applied as a coating e.g. using spray coating, dip coating, spincoating, painting, hot mold or any other conventional method. In an embodiment, the monomers for the zwitterionic polymer is polymerized directly onto the surface e.g. a silanized surface (not according to the invention).

Also disclosed are the zwitterionic monomers with formula I - XIII and their use for providing an anti-fouling coating and/or protein repelling surface.

The zwitterionic monomers with formula I - XIII is as follows:

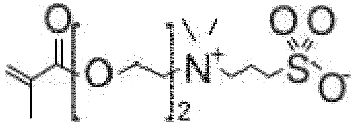

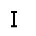

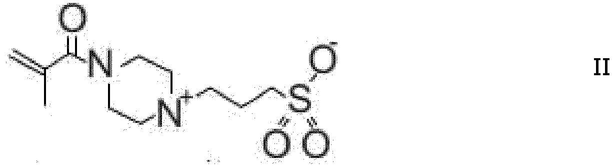

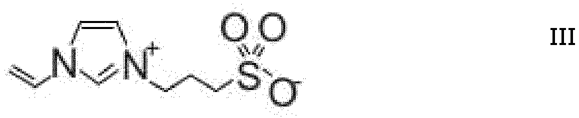

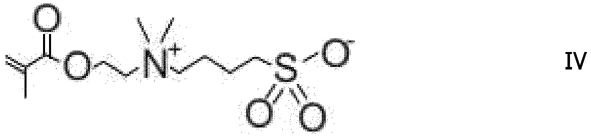

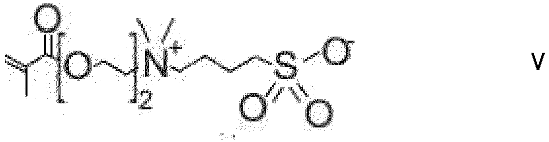

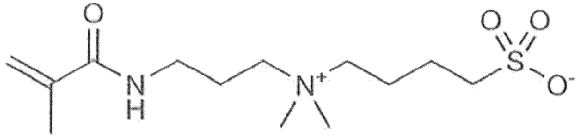

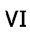

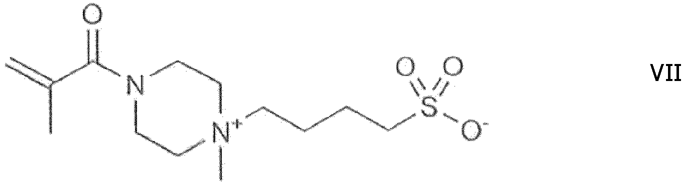

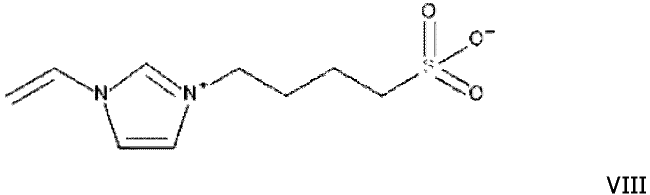

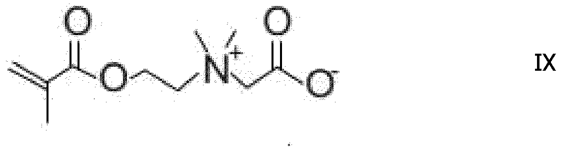

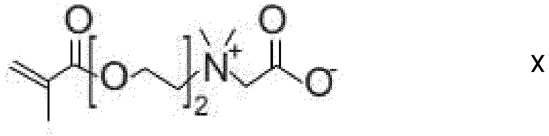

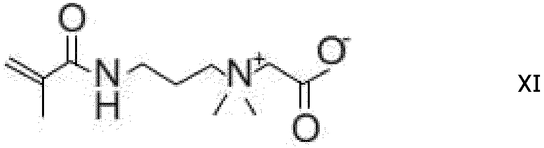

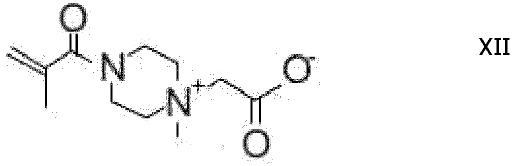

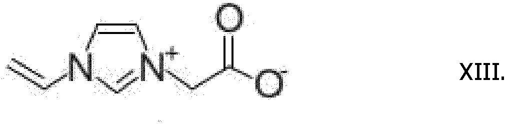

### Brief description of preferred embodiments and elements of the invention

The above and/or additional objects, features and advantages of the present invention will be further elucidated by the following illustrative and non-limiting description of embodiments and examples of the present invention, with reference to the appended drawings.

The figures are schematic and are not drawn to scale and may be simplified for clarity. Throughout, the same reference numerals are used for identical or corresponding parts.
Figures 1a and 1b are side views of examples of a medical tubular device of the invention.
Figures 2a-2d are side views of further examples of a medical tubular device of the invention.
Figures 3a-3c are side cross-sectional views of sections of body structure of examples of medical tubular devices of the invention. The sectional views are seen in cuts along the length of the body structures.
Figure 4 illustrates a setup for simultaneous treatment, e.g. loading and/or reacting of a luminal surface guest polymer domain and an external surface guest polymer domain of a medical tubular device of an embodiment of the invention.
Figure 5 show examples of anions and cations, which may form part of the zwitterionic moieties of a zwitterionic hydrogel of a medical tubular device of an embodiment of the invention.
Figure 6 show examples monomers, which may be used for the guest polymer domain of a medical tubular device of an embodiment of the invention.
Figure 7 show the spiropyran in its "spiro form" where it is substantially color-less and the spiropyran in its "merocyanine form" where it is purple.
Figures 8a and 8b show samples of medical tubular devices containing spiropyran in a first condition (after exposure to visual light) and a second condition (after exposure to UV light) respectively.

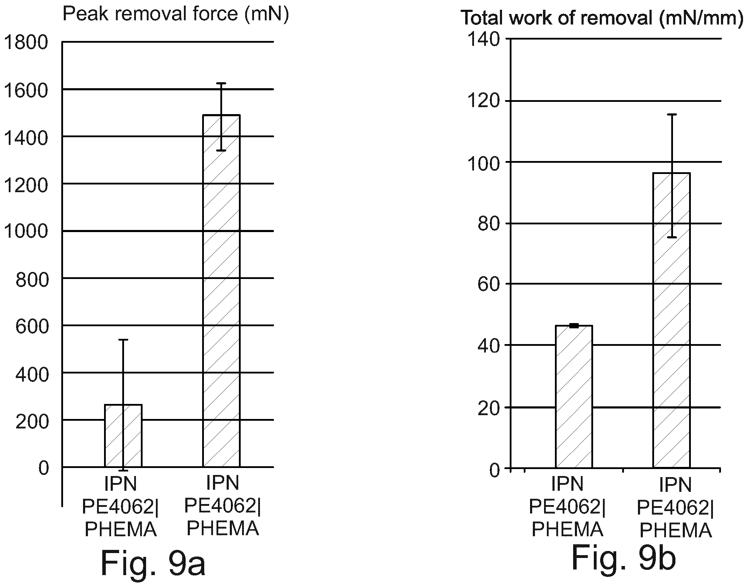

Figures 9a and 9b are curves showing the force required to remove samples with and without chitosan respectively, from a surface covered with an epithelial layer.
Figure 10 shows the back of a pig used to evaluate the risk of infection of the tubular medical device. Six pieces of medical tubular device loaded with a combination of minocycline and rifampicin are implanted on one side of the back. Six pieces of commercial state-of-the-art vascular graft (Propaten CBAS heparin coated, Gore) is implanted on the other side. All pieces are challenged with a load of bacteria. After two weeks, the implants are collected and the level of infection is determined/evaluated.
Figure 11a and 11b shows the implanted site after reopening after 14 days for the new medical tubular device and Gore's propaten CBAS heparin coated graft respectively.
Figure 12 shows the quantification of biofilm bacteria (*S*. *aureus* ATCC 29213) on IPN and Gore's propaten CBAS heparin coated graft materials (left) and in the surrounding subcutaneous tissue near the implanted materials (right).
Figure 13 shows the quantification of biofilm bacteria (*S*. *aureus* ATCC 29213) in IPN and Gore's propaten CBAS heparin coated graft material as function of bacteria challenge.
Figure 14 shows the analysis of functional Taxol release from the new tubular medical device loaded with Taxol. Graph indicates cell proliferation (EA.hy926 endothelial cell culture) after exposure to release media from 10mm sections of Taxol-loaded IPN grafts (•) or unloaded IPN controls (■). After 21 weeks, grafts still exhibit release of Taxol at levels that completely inhibits endothelial cell growth.
Figure 15a and 15b show the explant of Gore's propaten CBAS Heparin vascular graft and new medical tubular device, respectively, after implanted as a vascular bypass graft in the carotid artery of sheep for 6 months.
Figure 16 shows a methacrylic monomer with a protecting group protecting a functional moiety, the protecting group is preferably fluorenylmethyloxycarbonyl (FMOC) and/or the protected functional moiety preferably comprises a -NH₂ group.

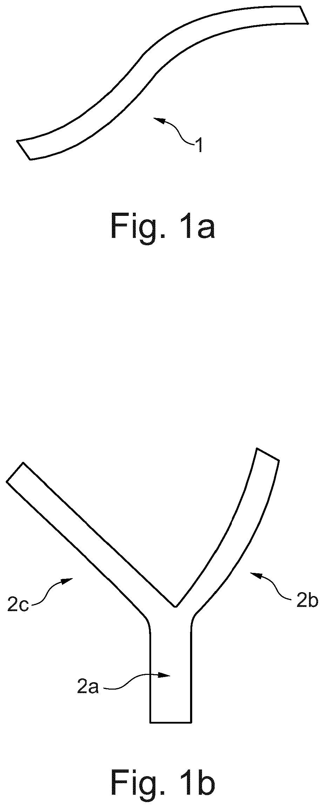

Figure 1a illustrates an embodiment of a linear medical tubular device 1, which is suitable for use as a vascular graft. The medical tubular device consists in this embodiment of the body structure 1 and has one single lumen.
Figure 1b illustrates another embodiment of a branched (bifurcated) medical tubular device, which is suitable for use as a vascular graft. The branched medical tubular device comprises a first part 2a with a first lumen and branched parts 2b and 2c branching the lumen into two lumens.

In practice, the medical tubular device may have any desired number of branches. Preferably, the medical tubular device is a non-branched medical tubular device.

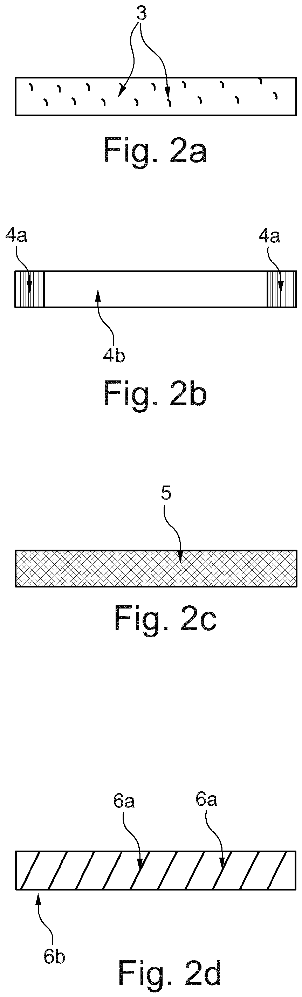

Figure 2a show a medical tubular device with a body structure having small anchor points 3 e.g. provided by small deformations in the external surface and/or by cilias shaped brushes of hydrogel (guest) polymer.

Figure 2b show a medical tubular device with a body structure 4b having cuffs 4a arranged at its external surface at each end of the body structure 4b. The cuffs may e.g. be as described above and may for example have the function of reinforcing the body structure e.g. for suturing.

Figure 2c show a medical tubular device with a body structure with a support net and/or a chitosan coating 5 onto its external surface.

Figure 2d show a medical tubular device with a helically support structure 6a applied onto the external surface 6b of the body structure.

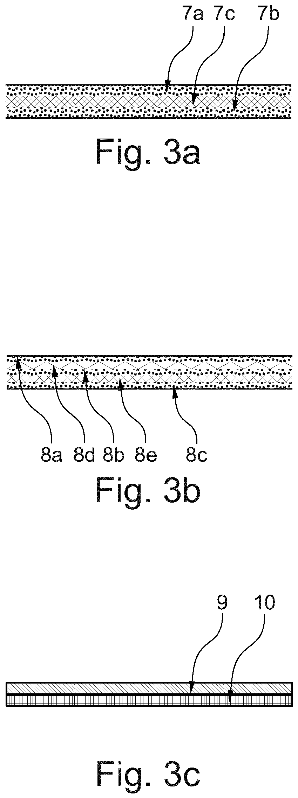

Figure 3a illustrates a body structure with a luminal surface guest polymer domain 7a and an external surface guest polymer domain 7b, where the luminal surface guest polymer domain 7a and the external surface guest polymer domain 7b are separated by a (intermediate) layer 7c of the host polymer without any guest polymer.

Figure 3b illustrates a body structure with a luminal surface guest polymer domain 8a, an intermediate guest polymer domain 8b and an external surface guest polymer domain 8c, where the guest polymer domains 8a, 8b, 8c are separated by layers 8d, 8e of host polymer without any guest polymer.

Figure 3c illustrates a body structure with a layered structure comprising an IPN layer 10 and a further, non-IPN layer 9, such as a PTFE layer. As explained above the body structure of embodiments of the invention may have several further layers, such as 2, 3, 4 or 5.

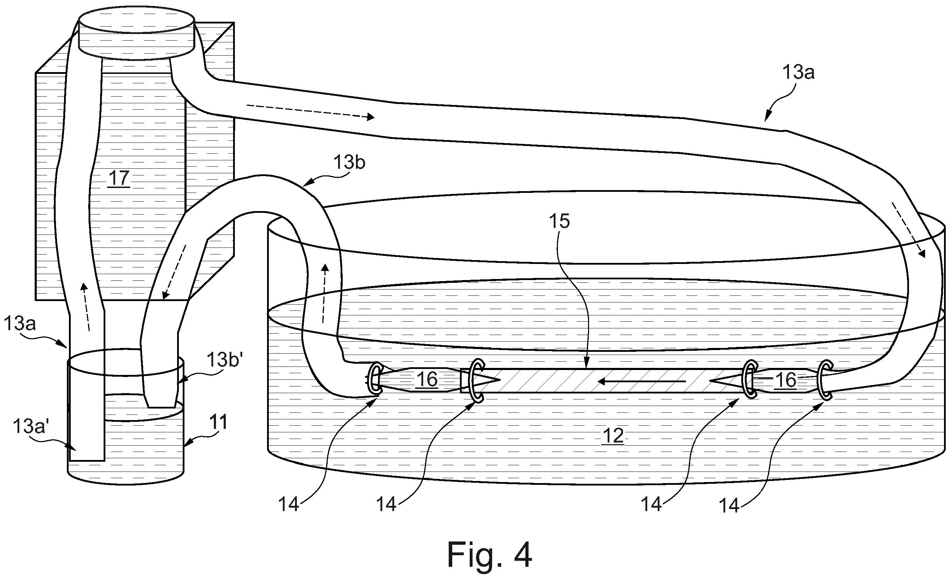

The setup shown in figure 4 comprises a first container 11 with a first treatment fluid (for treating the luminal guest polymer), a second container 12 with a second treatment fluid (for treating the external guest polymer) and a peristaltic pump 17.

A medical tubular device 15 of an embodiment of the invention is provided to have a luminal surface guest polymer domain and an external surface guest polymer domain e.g. as described elsewhere herein.

The medical tubular device is mounted to a first hose section 13a and a second hose section 13b via connecting studs 16 and hose clamps 14. The free end 13a' of the first hose section 13a is submerged into the first treatment fluid of the first container 11 and the free end 13b' of the second hose section is arranged to have outlet in the first container 11.

The first hose section 13a is connected to the peristaltic pump 17 to ensure that the first treatment fluid is pumped continuously or in steps from the first container 11, via the first hose section 13a and through the lumen of the medical tubular device 15 and further the treatment fluid is returned to the first container 11 via the section hose section 13b. The peristaltic pump 17 could naturally instead have been connected to the second hose section 13b'.

In a variation thereof, the first treatment fluid may be held steady within the tube for the treatment time.

At the same time, the medical tubular device 15 is submerged in the second treatment fluid of the second container 12.

The first and the second treatment fluids (11 and 12) are advantageously different from each other. The first and the second treatment fluids (11 and 12) may independently from each other be selected from a loading fluid comprising at least one loading drug for loading the respective guest polymer domains and a reaction fluid comprising at least one reagent adapted for treating the guest polymer, e.g. to chemically bind a drug to the guest polymer.

In an embodiment, the first treatment fluid (11) is a loading drug and the second treatment fluid (12) is a reaction fluid. In an embodiment, the first treatment fluid (11) is a reaction fluid and the second treatment fluid (12) is a loading drug. The drug(s) may be as described elsewhere herein.

In an embodiment, the first treatment fluid (11) is a loading fluid comprising Taxol for being loaded into the luminal surface guest polymer domain, e.g. to provide a gradient of Taxol concentration in the luminal surface guest polymer domain. In this or in another embodiment the second treatment fluid (12) is a loading fluid for loading anti-infective drug such as Rifampicin and/or minocycline.

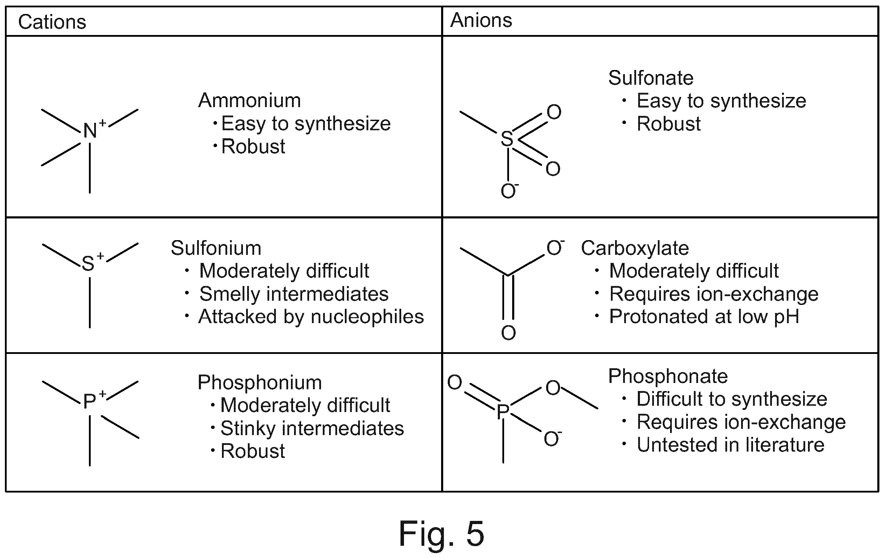

The examples of anions and cations of figure 5 are head moieties, which may be modified and/or combined to form a desired zwitterionic moiety and/or monomer.

The monomers shown in figure 6 comprises the zwitterionic monomers sulfobetanies, carbobetaines and phosphobetaines which may advantageously be applied for providing one or more guest polymer domain(s) and/or guest polymer surfaces of the medical tubular device.

The zwitterionic monomers are synthesized from respectively vinylimidazole, metacrylamides and methacrylate ester.

### Examples

### Example 1a - Synthesis of spiropyran-OH

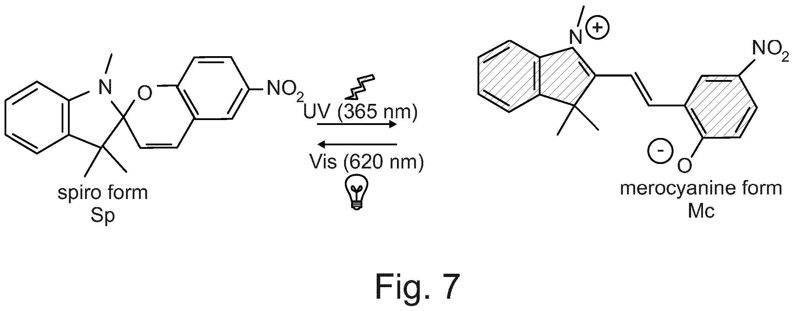

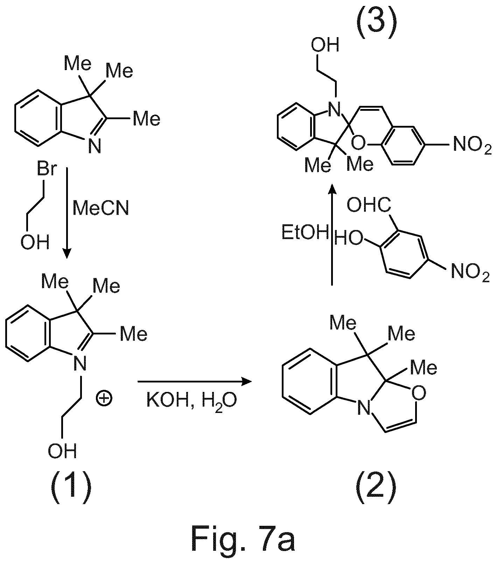

Synthesis of SP-OH was done in three steps (figure 7a), for each of the steps a HNMR examination was performed to confirm the structure. The analyze of HNMR spectrum was done by Bruker software. For each of the three steps, the HNMR of the samples shows all expected signals.

### Step 1: Synthesis of 1-(2-hydroxyethyl)-2,3,3-trimethyl-3H-indolium bromide (1 of figure 7a)

A solution of 2,3,3-trimethyl-3H-indol (2.61 g, 16 mmol) and 2-bromoethanol (2,46 g, 20 mmol) in MeCN (20 mL) was heated for 24 h under reflux and N₂. After cooling down to ambient temperature, the solvent was distilled off under reduced pressure. The residue was suspended in hexane (25 mL) and the mixture was sonicated and filtered. The resulting solid was crystallized from CHCl₃ (35 mL) to afford 1 (2.95 g, 69%) as a pink solid. The crystals were characterized with HNMR.

### Step 2: Synthesis of 9,9,9a-Trimethyl-2,3,9,9a-tetrahydro-oxazolo[3,2-a]indole (2 of figure 7a)

A solution of the compound of step 1 (2.93 g, 10 mmol) and KOH (0.92 g, 16 mmol) in H₂O (50 mL) was stirred at ambient temperature for 10 min and then it was extracted with Et₂O (3*20 mL). The organic phase was concentrated under reduced pressure to afford **2** (1.84 g, 88%) as a yellow oil and it was characterized with HNMR.

### Step 3: Synthesis of 2-(3,3'-dimethyl-6-nitro-3'H-spiro[chromene--2,2'-indol]-1'-yl)-ethanol (SP-OH) (3 of figure 7a)

A solution of 2-hydroxy-5-nitrobenzaldehyde (1.05 g, 6 mmol) and the compound of step 2 (0.87 g, 4 mmol) in EtOH (10 mL) was heated for 3 h under reflux and N₂. After cooling down to ambient temperature, the mixture was filtered. The resulting solid was washed with EtOH (2 mL) and dried to afford SP-OH (1.22 g, 81%) as a purple solid and it was characterized by HNMR.

### Example 1b - Synthesis of spiropyran monomer (SPMA):

The photoactive compound. SP is a photoactive compound which isomerizes from the hydrophobic (neutral) spiropyran state (so called closed form) to the hydrophilic (zwitterionic) merocyanine state (so called open form) under blue light irradiation (figure 7). As can be seen, the hydrophobic state (SP) is transparent while the hydrophilic state (MC) is purple. The photoisomerization of the SP is totally reversible and the hydrophilicity of the two isomers is significantly different.

Figure 7 show the spiropyran in its "spiro form" where it is substantially color-less in visible light and the spiropyran in its "merocyanine form" where it is dark purple.

The "merocyanine form" is hydrophilic, due to the generation of a zwitterion. The transformation between the "spiro form" and the "merocyanine form" is reversible and occurs by exposing the material to UV light and visible light respectively. This generates a hydrophilicity switch, where the materials hydrophilicity may be changed depending on the wavelength of the light that hits the material. Hydrophilicity switches may be utilized to trigger drug delivery, and/or attach/detach to cells.

The figure illustrates that the spiropyran can be switched from its spiro form to its merocyanine form by subjecting it to UV illumination e.g. at about 365 nm. After UV illumination, the spiropyran may slowly return from its merocyanine form to its spiro form or upon illumination with visible light, the returning to the spiro form may be faster.

The transfer between spiropyran and merocyanine form may also be induced by other impacts, like pH and/or mechanically.

### Example 2 - Medical tubular device with spiropyran

Samples of small medical tubular devices was produced. Tube shaped host polymer substrates of MED-4720 silicone elastomer, provided from Nusil was provided. The host polymer was loaded with monomers to provide a full IPN and the monomers was cross-linked e.g. as described elsewhere herein.

The hydrogel content in the medical tubular devices was > 30 % by dry weight.

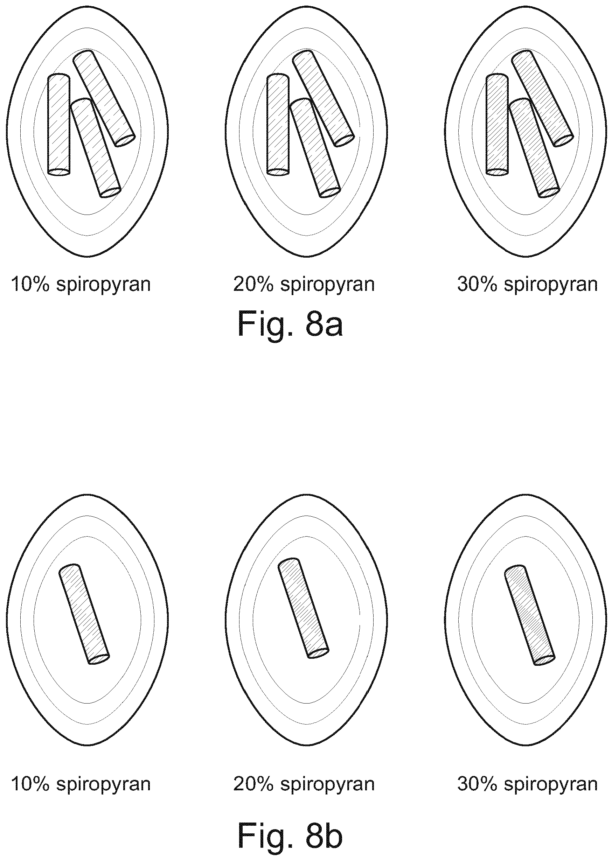

The co-monomer was 2-hydroxyethyl methacrylate (HEMA) and a minor percentage of spiropyran as shown in figure 8a and 8b. The percentage of spiropyran indicated is the percentage of monomers in the feed during monomer loading.

In figure 8a, 3 times 3 samples with varying spiropyran content of the samples of the medical tubular devices are shown after having being immersed in water to full saturation. The samples have a light brownish colour, which mean that the spiropyran is in its spiro form.

In figure 8b, 3 samples with varying spiropyran content (approx. 10, 20 and 30 % by weight of hydrogel) are shown after having being immersed in water to full saturation and subject to UV illumination. The samples are dark purple, i.e. it is in its merocyanine form.

This property may be very advantageous e.g. to trigger drug delivery, and/or attach/detach to cells. The change between SP and MC form changes the hydrophilicity of the polymer, due to the formation of a zwitterionic moiety.

In addition this property may be used to ensure that a medical tubular device has been correct implanted during surgery. Before closing up, the surgeon may illuminate the medical tubular device and control the fixation and tightness of the medical tubular device.

### Example 3 - Chitosan loading (not according to the invention)

Flat disc shaped samples (Ø 10 mm, thickness 2.0 mm) of silicone (PDMS) polymer was punched from an extruded PDMS strip delivered from Lebo Production (Lebo Production, Skogaas, Sweden) with the trade name "PE4062". 6 samples were arranged in a 16 ml reactor and impregnated with guest polymer PHEMA (poly(2-hydroxyethyl methacrylate)) using CO₂ to a pressure of 300 bar at 22 °C for 24 hours. The pressure was slowly released and 3 of the samples (blinds) were collected in airtight bags.

### The 3 remaining samples were loaded with chitosan in the 16 ml reactor

The reactor was filled with 10 ml of 10 mg/ml chitosan solution (100 g chitosan in 7.5 ml EtOH, 2 ml H₂O and 1 ml acetic acid).

The reactor was pressurized using CO₂ to a pressure of 300 bar at 22 °C for 24 hours.

The pressure was slowly released and the 3 samples were collected in airtight bags.

Experimental setup for testing of bioadhesion: Bioadhesion was measured on a Texture Analyzer Texture (TA:.XT.plus, TA Instruments). Bioadhesion was simulated on absorbent paper wetted with a 2 % porcine gastric mucus (Sigma) solution in phosphate buffer pH 6.8. Discs were pressed against the mucus-wetted paper for 180 s before removed. Adhesion was measured as peak force necessary to remove discs and total work to remove disc (area under the curve).

The results are shown in figured 9a and 9b.

### Example 4 - Chitosan detection (not according to the invention)

Samples obtained from example 3 are each stained with 0.5ml 0.1% (w/v) Fluorescent Brightener 28 (FB28 Sigma F3543) dissolved in 0.5M Tris·HCl, pH 9.

Surplus of stain is removed by washing three times. Each wash consists of 0.5 ml of water.

Devices not treated with chitosan (Blind) are used as references for the evaluation of fluorescence related specifically to the presence of chitosan in devices that have been treated.

Fluorescence is monitored by placing devices on a UV transilluminator (302nm) and documenting the emission with CCD pictures (G:BOX, Syngene). These pictures can be quantitatively accessed for estimation of relative content or with reference to devices containing known amounts.

### Example 5 - Attaching chitosan to silicone (not according to the invention)

A number of chitosan foams is produced by freeze-drying chitosan gels with concentration of chitosan of about 0.5 %, 1 %, and 2 %.

The chitosan foams have thickness between 0.5 and 1 mm. Samples are cut into 1cm² pieces.

Samples of standard silicone are cut into 1cm² pieces in order to match the sizes of KitoZyme chitosan foam preparations.

Foams is attached to a number of the standard silicone slabs by using Sylgard 184 (Dow Corning, Diatom) for attaching. Uncured Sylgard 184 is applied onto the surface of cast standard silicone using a brush.

Chitosan powders of particle size below 80 µm is provided. The powder is attached to the surface of a number of silicone samples by dipping the cast standard silicone samples into uncured Sylgard 184 silicone and then bring the surfaces in contact with the powder.

It will be seen that powdered chitosan is easily applied onto the surface of cast standard silicone and appear to be surface accessible. Chitosan remain attached after overnight incubation. This allow all edges to be covered.

It will further bee seen that the chitosan foam with big pores allow for efficient attachment onto the silicone surfaces. The chitosan foam remains attached after overnight incubation in water. The foam appears to become embedded in the Sylgard elastomer and is expected to be less available to cell interaction than the powdered chitosan.

Further it will be seen that also chitosan with smaller pores allow for efficient attachment onto the surfaces of cast silicone. The chitosan foam remains attached after overnight incubation in water. The foam appears to become completely embedded in the Sylgard elastomer and is expected to have far less chitosan available for cell interaction than the powdered chitosan.

### Example 6 - Pig test (not according to the invention)

A provoked surgical site infection (SSI) animal (pig) model was established. To compare Interpenetrating polymer network (IPN) vascular graft material and currently used heparin coated ePTFE material (Gore-Tex) for infection resistance, by direct contamination with *Staphylococcus Aureus* in a porcine model.

IPN patches (Host polymer: MED-4720 silicone elastomer/ Guest polymer: PHEMA-co-PEGMEA - with 23 % hydrogel) was loaded.

Loading was done 2 weeks prior to experiments. The loading was done in two steps. In step 1, the patches were loaded in 10 mg/ml rifampicin in 96% ethanol for one week. In step 2, the patches were loaded in a 10 mg/ml minocycline solution in MilliQ water saturated with rifampicin (approx. 2.5 mg/ml) for one week. On the day of experiment, the patches were washed by incubation in PBS at room temperature for 15 minutes prior to insertion.

The direct comparison experiment was conducted on six female Danish Landrace pigs weighing about 80 kg. On the right side of the pigs, six Gore-Tex Propaten CBAS Heparin coated vascular graft patch (20.0x10.0x0.6mm) were implanted and on the left side, six IPNs of same dimensions were implanted. These patches were inoculated with 10⁶ *Staphylococcus aureus.* The implants were removed after 14 days.

In a first run, the dose of *S*. *aureus* was changed from 10³ to 10⁷. It was found that the optimal challenge was 10⁶ bacteria. Therefore, a second run was made with 10⁶ bacteria. The second run was conducted on two female Danish Landrace pigs weighing about 80 kg.

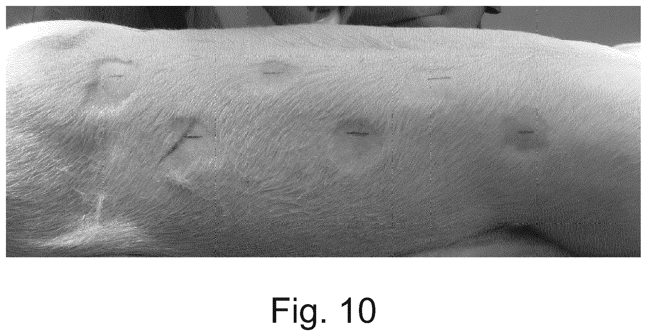

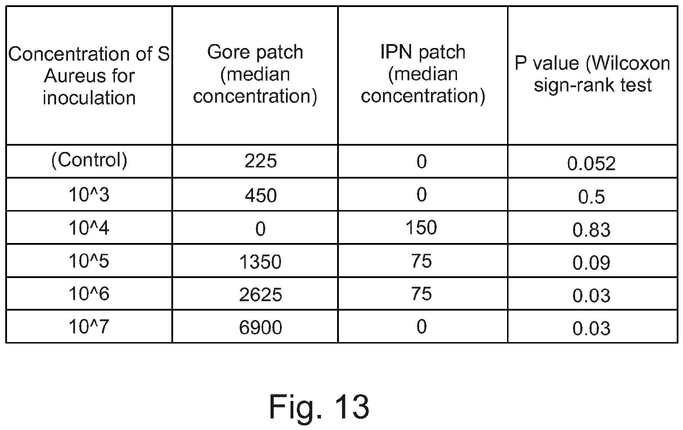

The patches were analyzed for concentration of *S. aureus* and the result is shown in figure 13. It can be seen that there is much less infection at the IPN patches than at the Gore patches
Figure 10 shows the right side of one of the pigs with the markings showing where the patches are implanted.

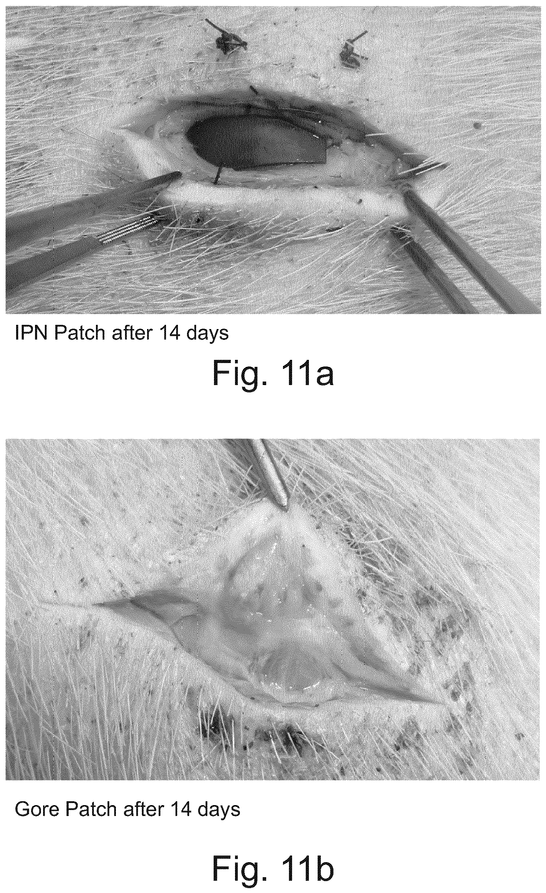

Figure 11a show an IPN Patch after 14 days, where the patch initially was inoculated with 10⁶ *S. Aureus.*
Figure 11b show a Gore Patch after 14 days, where the patch initially was inoculated with 10⁶ *S. Aureus.*

It can be seen that the IPN patch suppresses the infection. The P value was determined on the hypothesis that the risk of infection was not dependent on the patch used. Since the P value in very low there is a clear indication that this hypothesis is not correct and that there in fact is a much lower risk of infection using the IPN patch relative to the Gore patch. Rifampicin and minocycline loaded IPN has been tested in one additional round of a porcine subcutaneous infection model and compared with state-of-the-art Gore graft material. The second run was conducted on two female Danish Landrace pigs weighing about 80 kg with a bacteria challenge/load of 10⁶ bacteria in each surgical site wound.

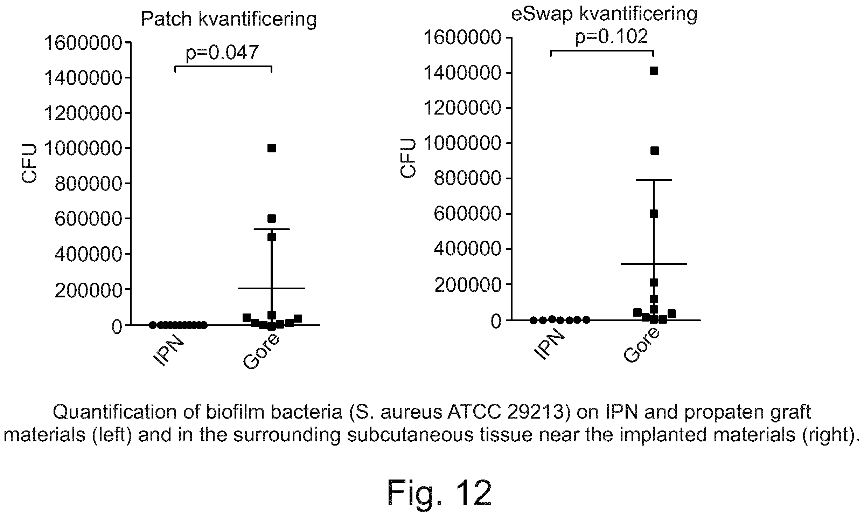

The results (cf. figure 12) showed again better performance for the IPN material, with approx. 10³ times reduction in biofilm bacteria associated with the IPN material compared to Gore's Propaten.

### Example 7 - Loading with taxol

A number of medical tubular device were produced:
IPN host polymer: Nusil MED-4720 silicone elastomer extruded into thin tubing by Vesta (USA). The tubing of host polymer had the following dimensions: ID 5.0mm, wall thickness 0.75 mm.
IPN guest polymer: copolymer of PHEMA, PEGMEA-480 (Poly(ethylene glycol) methyl ether acrylate average Mn 480) and CK1573 (vinylimidazole butyl sulfonate).
Hydrogel content (dry) was 41%.
A sample of the medical tubular devices (unloaded medical tubular device) was packed in an airtight bag.
IPN graft was loaded in taxol stock solution for 5 days. "Paclitaxel "Fresenius Kabi": 6 mg/ml PTX in 50:50 solution of ethanol and ricinus oil. http://pro.medicin.dk/Medicin/Praeparater/5984
After loading, the medical tubular devices were packed in airtight bags.

### Example 8 - release

The unloaded medical tubular device of example 7 (Control) and one taxol loaded medical tubular device of example 7 were used.

The control sample and the taxol loaded sample were each immersed in an aqueous cell growth medium. After each week a small sample of each aqueous cell growth medium are withdrawn and are added into respective wells and about 25,000 endothelial cells (EA.hy926 endothelial cell culture) are added to each well. After two days, the cells are counted.

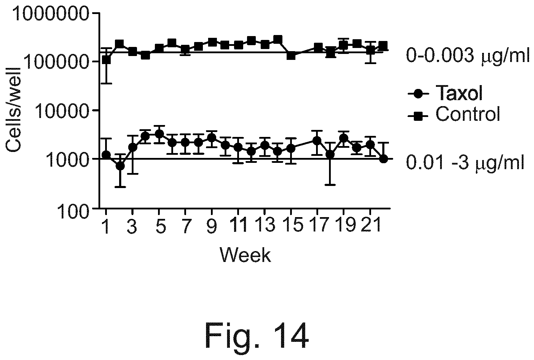

The results are shown in figure 14. It can be seen that after 21 weeks, the IPN graft still exhibit release of Taxol at levels that completely inhibits endothelial cell growth.

### Example 9 - Sheep test

We identified a special race of sheep bred at the south west coast of Jutland (Denmark), which are relatively calm and easily handled postoperatively.

Five sheep were selected for the test. An end-to-side bypass operation of the common carotid artery bilaterally in each sheep was performed, using a Gore^{®} Propaten CBAS heparinized graft and a taxol loaded medical tubular (IPN) device of example 7 as graft at respective sides of the sheep. The sheeps were monthly inspected by duplex scanning.

After 6 month, the grafts were removed.

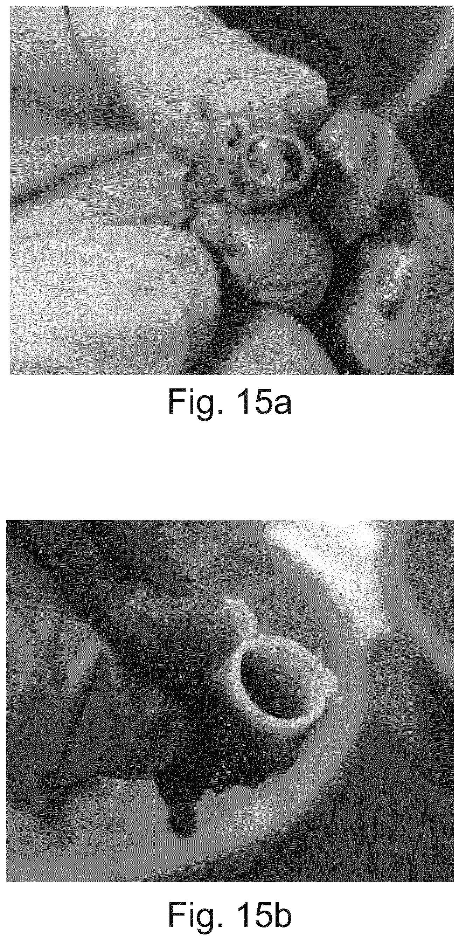

Figure 15a show one of the Gore^{®} Propaten CBAS heparinized graft. Figure 15b show one of the taxol loaded medical tubular IPN device of an embodiment of the invention.

It can be seen that the Gore^{®} Propaten heparinized graft appears to be completely closed, whereas the IPN devices appears to be fully open and with no signs of hyperplasia.

### Example 10 - FMOC procedure

Four samples each 1 cm x 4 cm x 1 mm were used:

| | | |
|---|---|---|
| Sample A | Silicone | PMDS |
| Sample B | IPN | Host: PMDS; Guest: PHEMA hydrogel |
| Sample C | IPN | Host: PMDS; Guest: PHEMA-co-PEGMEA hydrogel |
| Sample D | IPN | Host: PMDS; Guest: PHEMA-co-PEGMEA-co-CK1594 hydrogel |

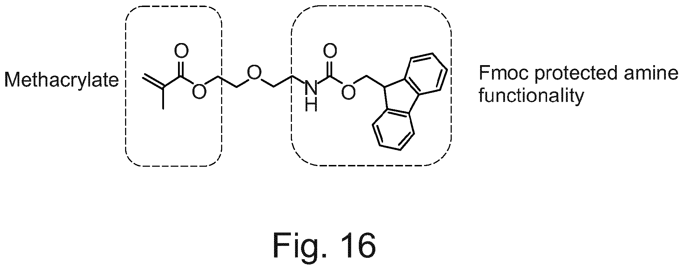

The CK1594 monomer forming part of the hydrogel guest polymer in sample D is a monomer with an FMOC protected amine as shown in figure 16.

The samples were placed in a 4 compartment wire-mesh basket and immersed into 25 ml beakers containing the reagents:
Deprotection: 20% piperidine in DMF for 2x2 min.
Coupling: 0.38 mmol HBTU, 0.37 mmol linker, 0.5 mmol DIPEA in 20 ml DMF.
Capping: 20% acetic anhydride in MeOH for 2 hours.
FITC (Fluorescein isothiocyanate) tagging: 0.1 mg/ml FITC in DMF for 2 hours.
Washing: Subjects were rinsed with MeOH and washed in DMF for at least 3x10 min between and after reactions.
Storage: Subjects were stored in DMF between reactions.

FITC has excitation and emission spectrum peak wavelengths of approximately 495 nm/519 nm, giving it a green colour. The samples were examined and the following was observed:

| | |
|---|---|
| Sample A | No sign of FITC |
| Sample B | No sign of FITC |
| Sample C | Very weak sign |
| Sample D | Clear strong sign of FITC |

The weak signal in sample C may be explained by absorption of FITC in the hydrogel. However, the signal is very weak compared to the signal of sample D, indicating that the signal observed in sample D is from attaching/reacting FITC to the IPN.

### Example 11 - Heparin grafting

A medical tubular IPN graft (OD 5 mm, ID 3 mm) comprising a host polymer of PMDS and a guest polymer forming at least a luminal surface guest polymer domain of PHEMA-co-PEGMEA-co-CK1594 hydrogel is produced (same method as sample D from example 10).

The IPN graft is arranged to be contacted with fluids at its luminal surface as shown in the setup of figure 4, but without the second container.

The luminal surface is reacted with deprotection fluid, coupling fluid and capping fluid following the scheme of example 10. Thereafter the luminal surface is reacted with heparin.

Heparin entities containing free terminal aldehydes of US2011064781 (Examples 1 through 6) is dissolved in DI water, pH 3.9 together with NaCl (approximately 0.5 g heparin and 2.95 g NaOH per 100 ml).

The luminal surface is contacted with the heparin solution at a temperature of 50 to 60 °C for 1-2 hours.

Thereafter the IPN graft is washed in water.

Further scope of applicability of the present invention will become apparent from the description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Example 12 - Zwitterionic IPNs

A number of zwitterionic IPN samples was prepared using zwitterionic monomers as listed in Fig. 6.

Silicone samples (tubings with outer diameter (OD): 5.10mm, inner diameter (ID): 4.10mm, thickness:0.50mm; approx. 35 g) were organized in a stainless steel grid and placed in a one-litre reactor (1L, BC-2, HiP, Pennsylvania, USA) on top of a 4 cm magnet bar (for stirring). Approximately 100 mL HEMA, 100 mL PEGMEA, 5 g zwitterionic monomer, 6 mL EGDMA, 50 mL 0.2M DEPDC in hexane, 70 mL EtOH and 70 mL THF (tetrahydrofuran) were mixed for 20 min in a separate beaker. The solution was then added to the reactor and the reactor was closed with the corresponding bolts with a torque of 125 ft-lb. The reactor was placed on top of a magnet stirrer (Ret Basic, IKA, Germany). The reactor was wrapped in a custom made heating jacket. Inlet and outlet was placed on top of the reactor, however inlet tubing was let to the bottom of the reactor for proper mixing and outlet tubing was let to a waste container. All tubing was 1/16" with an internal diameter of 0.03" (15-9A1-030, HiP, Pennsylvania, USA). A pressure transmitter was connected to the inlet. Pressure and temperature were monitored and logged through a custom made LabView program. A Thar P-50 electrical driven high-pressure pump from Thar Designs Inc. USA is applied for assuring the operation pressure. The pump was equipped with a heat exchanger and was supplied with cooling water at 5°C. CO₂ was added to the reactor to a pressure of 300 bar at 40°C for approx. 16 h. During this time monomers diffused into the swollen silicone, polymerize and cross-link. Then the pressure was lowered through the outlet tubing to the waste container.

Depressurizing the reactor takes between 30 - 60 minutes. Subsequent the reactor was first cleaned with ethanol and subsequently with demineralized water. The produced IPN samples were collected and excess polymer was removed by rinsing in tap-water. Excess monomer and uncrosslinked polymer were extracted by placing the IPN samples in 96% EtOH for 1 week. Then the IPN samples were dried until constant weight before the hydrogel content was determined by weighing.

The produced samples are listed in table 1

**Table 1.**

| Name | Zwitterionic monomer | Zwitterionic monomer | Hydrogel content W/W% |
|---|---|---|---|
| | No. (Fig. 6) | | |
| 1582-G9 | CK1582 | 3-[dimethyl(2-{2-[(2-methylprop-2-enoyl)oxy]ethoxy}ethyl)azaniumyl]propane-1-sulfonate | 29.77 |
| 1578-G9 | CK1578 | 1-methyl-4-(2-methylprop-2-enoyl)-1-(4-sulfonatobutyl)piperazin-1-ium | 30.10 |
| 1572-G9 | CK1572 | 1-Ethenyl-3-(3-sulfonatopropyl)-1H-imidazol-3-ium | 31.28 |
| 1583-G9 | CK1583 | 4-[dimethyl({2-[(2-methylprop-2-enoyl)oxy]ethyl})azaniumyl]butane-1-sulfonate | 27.93 |
| 1591-G9 | CK1591 | 4-(2-methylprop-2-enoyloxymethoxymethylammonio)butane-1-sulfonate | 51.07 |
| 1584-G9 | CK1584 | 4-{dimethyl[3-(2-methylprop-2-enamido)propyl]azaniumyl} butane-1-sulfonate | 35.39 |
| 1586-G9 | CK1586 | 1-methyl-4-(2-methylprop-2-enoyl)-1-(4-sulfonatobutyl)piperazin-1-ium | 17.03 |
| 1573-G9 | CK1573 | 1-Ethenyl-3-(4-sulfonatobutyl)-1H-imidazol-3-ium | 46.08 |
| 1581-G9 | CK1581 | 1-carboxy-N,N-dimethyl-N-(2'-methacryloyloxyethyl)methanaminium | 35.50 |
| 1589-G9 | CK1589 | 2-[dimethyl(2-{2-[(2-methylprop-2-enoyl)oxy]ethoxy}-ethyl)azaniumyl]acetate | 35.50 |
| 1588-G9 | CK1588 | 2-{dimethyl[3-(2-mehtylprop-2-enamido)propryl]-azamniumyl}acetate | 42.51 |
| 1585-G9 | CK1585 | 1-(carboxylatomethyl)-1-methyl-4-(2-methylprop-2-enoyl)piperazin-1-ium | 42.93 |
| 1587-G9 | CK1587 | 3-(carboxylatomethyl)-1-ethenyl-1H-imidazol-3-ium | 43.68 |
| 1573-G8 | CK1573 | 1-Ethenyl-3-(4-sulfonatobutyl)-1H-imidazol-3-ium | |
| 1573-G10 | CK1573 | 1-Ethenyl-3-(4-sulfonatobutyl)-1H-imidazol-3-ium | |

The G7/G8/G9 part of the name indicates the silicone used.

G7: Nusil Med 4020, Durometer: 25 Type A (extruded tubing (Vesta, US) L=2m, wall=0.5mm, ϕ(inner)=4.1mm.

G9: Nusil Med 4720, Durometer: 25 Type A (extruded tubing (Vesta, US) L=2m, wall=0.75mm, ϕ(inner)=5mm).

G10: Nusil Med 4027, Durometer: 30 Type A (extruded tubing (Vesta, US) L=0.5m, wall=0.75mm, ϕ(inner)=5mm).

### Example 13 - Thrombin-antithrombin complex (TAT) level.

A number of the IPN samples from example 12 was tested for blood compatibility. The zwitterion-based IPNs were evaluated using enzyme-linked immunosorbent assay (ELISA) that specifically measures the thrombin-antithrombin (TAT) and complement split-product C3c as sensitive markers for coagulation and complement activation, respectively. Both markers are included in the ISO standard 10993-4 for evaluation of biomaterials.

Plasma samples incubated on zwitterionic IPNs and samples of silicone (reference samples) were analyzed for Thrombin-Antithrombin (TAT) levels using ELISA. Maxisorp plates were coated with 2.0 µg/ml anti-TAT monoclonal antibody (HYB 14-22) O/N at 4 °C. The next day, the plates were washed three times in PBS with 0.05% Tween-20 and blocked for 15 min. Samples of human plasma were diluted to 1:320, and a serum pool diluted from 1:100 to 1:1024 was used as standardized samples using dilution in PBS + 0.1% BSA + aggregated IgG +10 mM EDTA. Samples were incubated for 1 hour at room temperature and washed as previously. The biotinylated secondary antibody 230-01 was diluted to 1:500 and incubated for 1hour. Each plate was washed and streptavidin-conjugated HRP was added at 1:4000 and incubated for 30 min followed by 3x wash. Color was allowed to develop for 15 min using OPD/H₂O₂ solution and stopped with 100 µl of 1M H₂SO₄. The plates were measured at 490 nm using 650 nm as reference, and then quantified for TAT concentrations using the SoftMax Pro software.

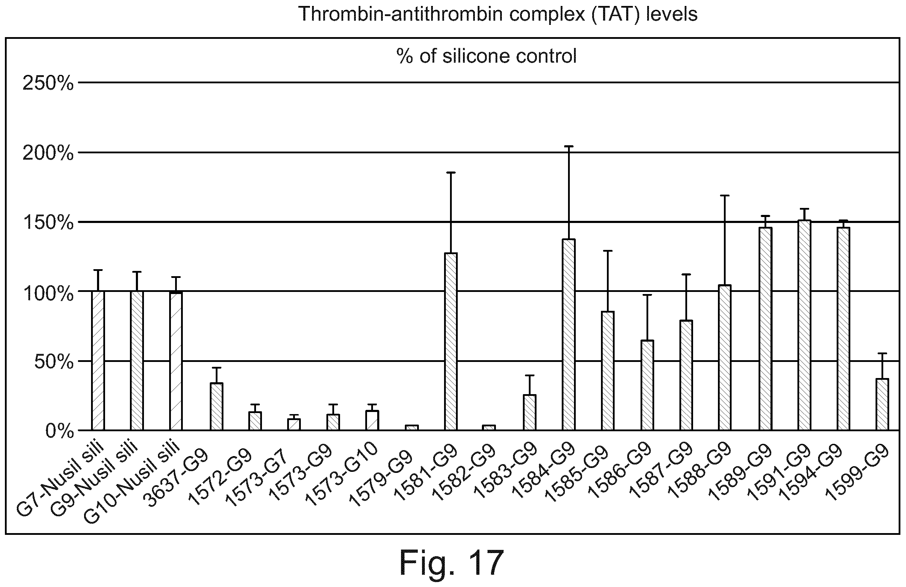

The results are shown in figure 17.

### Example 14 - zwitterionic IPNs with different amount of zwitterionic monomer

A number of zwitterionic IPN samples was prepared using three different amount of the zwitterionic monomer CK1573. The zwitterionic IPN samples were produced as described in example 12, but using respectively 2g, 5g, and 10g zwitterionic monomer.

Six samples of each type (2g, 5, and 10 g zwitterionic monomer) were tested as described in example 13 using defibrinated plasma and citrated plasma respectively. Each group was compared to the silicone control using Dunnett's test for significance. P-values <0.05 were defined as significant, and shown as *P<0.05, **P<0.01, ***P<0.001.

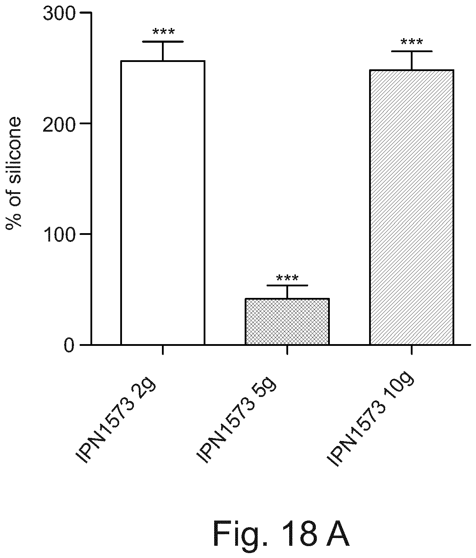

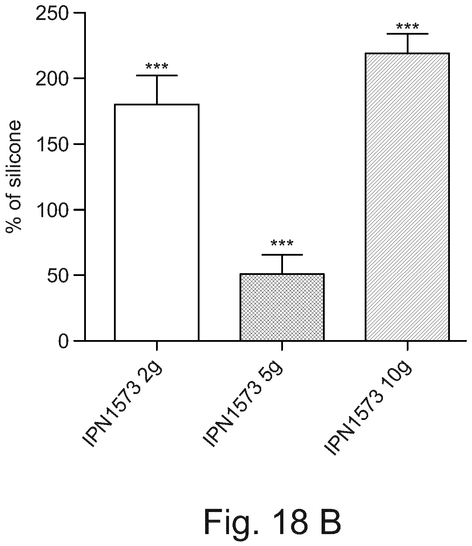

The results are shown in figure18A (defibrinated plasma) and figure 18B (citrated plasma)

### Example 15- Zwitterionic polymer coating suppress protein adsorption

Two test containers A and B, each with a volume of 5 L was provided. The containers have an inner surface of aluminum.

The inner surface of container A is coated with a zwitterionic containing hydrogel coating. The zwitterionic containing hydrogel coating is provided from HEMA, PEGMEA and zwitterionic monomer 1573 in the relative molar amounts 35:10:1.

The container A was first subjected to a silane coupling agent surface treatment. The inner surface of the container was cleaned and a solution of 3-methacryloxypropyl trimethoxy silane (2 wt% v/v in an acetic acid/water/ethanol mixture, 0.01: 1:4) was applied onto the inner surface. After 2h the inner surface was rinsed with ethanol and dried.

The hydrogel coating was prepared by monomer polymerization directly onto the silanized solid substrates. The surface was subjected to an aqueous precursor solution of the monomers and subjected to UV photopolymerization (365 nm wavelength, 30 mW cm⁻²) for 30 min. Thereafter the container was filled with pure water 3 days until they reached swelling equilibrium.

The precursor solution comprises HEMA, PEGMEA and zwitterionic monomer 1573 in the relative molar amounts 35:10:1 and 0.5 mol% of cross-linker, and 0.5 mol% of an initiator.

3 L of fresh milked cow milk is added into each container and hold there for 3 hours a 5 °C Thereafter the containers were emptied and cleaned by spraying tap water (20 °C) onto the surfaces for 1 minute. Thereafter the surfaces were examined for adsorbed protein.

The amount of protein remaining on the inner surface of container A is highly reduced compared to the amount of protein remaining on the inner surface of container B.

### Example 16 - Gradient loading of taxol in inner lumen and luminal surface of a medical tubular device (a graft)

Where there may be risk of cell growth inside the lumen of the medical tubular device, it is desired that the anti-proliferative drug is located at least as a part of the luminal surface.

The graft was sterilized by sonication of two turns in 70 vol.% ETOH for 5 min. The graft was then dried over the weekend in sterile Petri dishes in sterile benches.

The graft was mounted vertically on a stand with clamp at the bottom and with pipette tip at the top mounted on with strips.

Pure taxol solution (6 mg / ml taxol in a 50:50 mixture of ethanol and castor oil, Fresenius-Kabi) was filled into the lumen of the graft and air bubbles were massaged out during filling.

It was charged for 2½ hours in a sterile bench. The graft was emptied and rinsed vigorously inside using 100 ml of 70 vol.% ETOH applied with rod pipette.

The exterior and interior were rinsed from the top with approximately 300 ml of 70 vol.% ETOH.

The graft was briefly dried on a tripod and then cut into 10-14 cm graft pieces, which were placed in sterile autoclave bags, which were then sealed. The graft pieces are now ready for use in surgery.

### Example 17 - Synthesis of 1-ethenyl-3-(4-sulfonatobutyl)-1H-imidazol-3-ium (CK1573)

A 1L, 3-necked round-bottomed flask was fitted with mechanical stirrer, contact thermometer and N2-bubbler. The flask was charged with 1,4-butane sultone (0.48 mol, 63.4 g), 1-vinylimidazole (0.40 mol, 37.6 g) and MeCN (250 mL). The reaction was stirred at 60 °C for 72 hours in the dark. The reaction was diluted with MeCN (125 mL) and cooled to room temperature with stirring to keep the precipitate suspended. The reaction mixture was filtered and the reaction flask was rinsed with MeCN to transfer the remaining precipitate to the filter. The solids were washed with MeCN (250 mL) and MTBE (2 × 100 mL) and dried under reduced pressure for 24 hours to give the title product 87.5 g (95 %) as a white solid. 1H NMR (300 MHz, D2O) δ 7.80 (d, J = 2.2 Hz, 1H), 7.63 (d, J = 2.1 Hz, 1H), 7.16 (dd, J = 15.6, 8.7 Hz, 1H), 5.82 (dd, J = 15.6, 2.8 Hz, 1H), 5.45 (dd, J = 8.7, 2.8 Hz, 1H), 4.32 (t, J = 7.1 Hz, 2H), 3.02 - 2.94 (m, 2H), 2.14 - 2.02 (m, 2H), 1.85 - 1.72 (m, 2H). 13C NMR (75 MHz, d2o) δ 134.2 (t, 1JC-D = 34 Hz), 128.1, 122.7, 119.5, 109.3, 49.9, 49.2, 27.9, 20.8.

One imidazole proton is missing due to deuterium exchange which also causes the 13C triplet at 134.2 ppm.

### Example 18: Synthesis of 1-ethenyl-3-(3-sulfonatopropyl)-1H-imidazol-3-ium (CK1572)

A 1L, 3-necked round-bottomed flask was fitted with mechanical stirrer, contact thermometer and N2-bubbler. The flask was charged with 1,3-propane sultone (0.48 mol, 58.6 g), 1-vinylimidazole (0.40 mol, 37.6 g) and MeCN (200 mL). The reaction was stirred at room temperature for 72 hours in the dark. The reaction was diluted with MeCN (125 mL) to keep the precipitate suspended. The reaction mixture was filtered and the reaction flask was rinsed with MeCN to transfer the remaining precipitate to the filter. The solids were washed with MeCN (250 mL) and MTBE (2 × 100 mL) and dried under reduced pressure for 24 hours to give the title product 86.3 g (>99 %) as a white solid. 1H NMR (300 MHz, D2O) δ 7.82 (d, J = 2.2 Hz, 1H), 7.66 (d, J = 2.2 Hz, 1H), 7.17 (dd, J = 15.6, 8.7 Hz, 1H), 5.83 (dd, J = 15.6, 2.8 Hz, 1H), 5.46 (dd, J = 8.7, 2.8 Hz, 1H), 4.44 (t, J = 7.2 Hz, 2H), 3.02 - 2.90 (m, 2H), 2.45 - 2.28 (m, 2H). 13C NMR (75 MHz, D2O) δ 134.4 (t, 1JC-D = 34 Hz), 128.1, 122.7, 119.6, 109.4, 48.0, 47.1, 24.9.

## Claims

1. A medical tubular device comprising a body structure having an elongate hollow structure extending from a first end to a second end of the medical tubular device and having a luminal surface and an external surface, wherein said body structure having a continuous matrix of a host polymer matrix and comprises an interpenetrating polymer network (IPN) comprising the host polymer matrix and at least one hydrogel guest polymer domain of a guest polymer comprising a plurality of interconnected paths of the guest polymer, which is interpenetrating the host polymer matrix, wherein said host polymer matrix is a covalently cross-linked elastomer matrix, wherein said at least one hydrogel polymer domain comprises a luminal surface guest polymer domain comprising at least a part of the luminal surface, wherein a plurality of the paths of the guest polymer coincide and form at least a part of said luminal surface and wherein said luminal surface comprises zwitterionic moieties, said zwitterionic moieties being covalently bonded to said guest polymer.

2. The tubular device of claim 1, wherein said guest polymer comprises a zwitterionic hydrogel comprising a cross-linked network of polymerized monomers comprising of one or more type of monomers, preferably the zwitterionic monomers comprises at least one of sulfobetanies, carbobetaines, phosphobetaines, phosphocholines or any combinations thereof.

3. The tubular device of any one of the preceding claims, wherein the external surface comprises a surface at least partly of said guest polymer wherein a plurality of the paths of the guest polymer coincide and form at least a part of said external surface, and wherein said external surface comprises zwitterionic moieties, said zwitterionic moieties preferably being covalently bonded to said hydrogel (guest) polymer.

4. The tubular device of any one of the preceding claims, wherein the luminal comprises at least one covalent bonded drug, comprising a thrombosis deactivating drug(s), said thrombosis deactivating drug(s) preferably comprises heparin, EDTA, antibiotics, citrate and/or any combination comprising one of the mentioned thrombosis deactivating drugs.

5. The tubular device of any one of the preceding claims wherein said body structure comprises a releasable drug selected from an anti-proliferative drug, such as paclitaxel and/or rapamycin releasable at least via said luminal surface and an anti-infective drug such as rifampicin and/or minocycline releasable via at least said external surface.

6. The tubular device of any one of the preceding claims, wherein the host polymer comprises a cross-linked elastomer, preferably selected from a thermoplastic elastomer (TPE), a polyolefin elastomer (POE), a polyurethane (PU), a rubber, thermoplastic polyurethane (TPU), a silicone elastomer or any combinations comprising one of the before mentioned elastomers, preferably the host polymer comprises silicone elastomer (polysiloxanes).

7. The tubular device of any one of the preceding claims, wherein the host polymer has a shore A hardness of from about 15 to about 70, a tear strength of at least about 25 kN/m and a stress at 200% elongation of at least about 0.3 MPa, such as at least about 0.4 MPa, such as at least about 0.5 MPa, such as at least about 0.6 MPa, such as up to about 3 MPa, such as up to about 2 MPa, such as up to about 1 MPa.

8. The tubular device of any one of the preceding claims, wherein the guest polymer is cross-linked, and is polymerized from monomers comprising at least one zwitterion and at least one of PHEMA, PEGMEA or vinylimidazole butyl sulfonate, preferably the hydrogel guest polymer comprises a copolymer polymerised from monomers comprising PHEMA, PEGMEA, vinylimidazole butane sulfonate and/or at least one of a sulfobetanine.

9. The tubular device of any one of the preceding claims, wherein the hydrogel guest polymer comprises a copolymer polymerized from monomers comprising at least one zwitterionic monomer, selected from a sulfobetaine, a carbobetaine, a phosphobetaine, or a phosphocholine and/or wherein the hydrogel guest polymer comprises a copolymer polymerised from monomers comprising a photoactive monomer, such as a spiropyran monomer, such as a spiropyran acrylate.

10. The tubular device of any one of the preceding claims, wherein the at least one guest polymer domain comprises a clot promoting domain and/or a cell proliferation promoting domain located at the external surface of the body structure and/or located at an intermediate guest polymer domain, which does not comprise the luminal surface or the external surface.

11. The tubular device of any one of the preceding claims, wherein the host polymer comprises two or more guest polymer domains, wherein the two or more guest polymer domains comprises a luminal surface guest polymer domain comprising at least a part of the luminal surface and an external surface guest polymer domain comprising at least a part of the external surface, wherein the two or more guest polymer domains are separated by a polymer portion essentially free of guest polymer, said polymer portion is preferably a portion of said host polymer matrix, which is essentially free of interpenetrating guest polymer.

12. The tubular device of any one of the preceding claims, wherein the body structure is a layered body structure comprising two or more layers, wherein at least one of said layers comprises or consist of the IPN, wherein at least one layer of the body structure is a polymer layer without an IPN, such as a polymer layer comprising or consisting of polytetrafluoroethylene (PTFE), expanded PTFE, Polyethylene terephthalate (PET), polyurethane (PU) and/or silicone elastomer, preferably the at least two layers of the two or more layers are interfacially bonded to each other by chemically bonds (grafting) and/or at least two layers of the two or more layers are anchored to each other, said anchoring of said at least two layers, preferably comprises a local adhesive bonding, sutures, staples, clips and/or pinholes.

13. The tubular device of any one of the preceding claims, wherein the tubular device comprises a tubular support structure located concentrically with the body structure, wherein the support structure comprises a helical wire and/or a cuff close-fitting to the external surface of the body structure and wherein the support structure being of a polymer material comprising collagen, nylon, polytetrafluoroethylene (PTFE), expanded PTFE, Polyethylene terephthalate (PET), polyurethane (PU) and/or silicone elastomer.

14. The tubular device according to claim 13, wherein the cuff comprises an IPN material loaded with one or more drugs, such as growth hormone, antifibrinolytic drug(s) clot promoting drug(s) and/or cell proliferation promoting drug(s).

15. The tubular device of any one of the preceding claims, wherein the body structure has an internal diameter of from about 2 mm to about 6 cm, a compliance (% inner diameter change per mmHg x 10⁻² in the range 80-200 mmHg) of from about 1 to about 10 and wall thickness up to about 1 cm, such as from about 0.01 mm to about 3 mm, such as from about 0.5 mm to about 1 mm.

16. The tubular device of any one of the preceding claims, wherein the medical tubular device is an implantable tubular device, such as a dialysis graft, a stent, a vascular graft, a stent graft or a Thoraflex hybrid device.

17. The tubular device of any one of the preceding claims, wherein the medical tubular device is a dialysis tube, a feeding tube, such as a gastrostomy tube, a venous catheter, such as a central venous catheter (CVC line) or a peripherally inserted central venous catheter (PICC line).

18. The tubular device of any one of the preceding claims 2-17, wherein the at least one zwitterionic monomer is selected from zwitterionic monomer with formula I - XIII

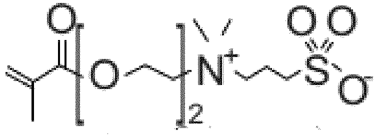

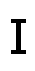

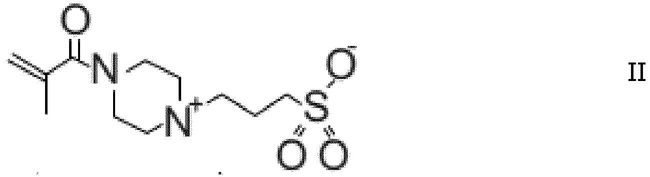

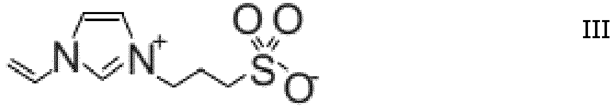

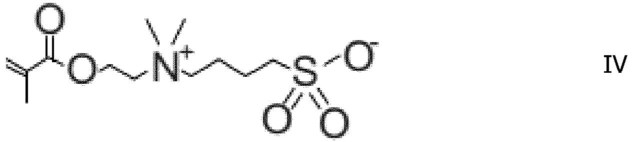

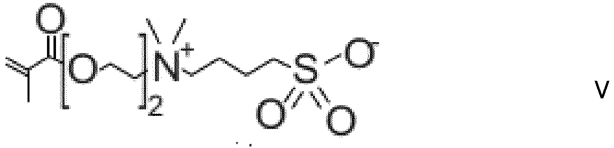

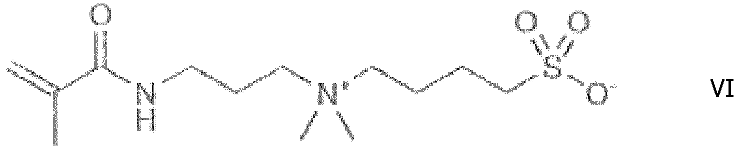

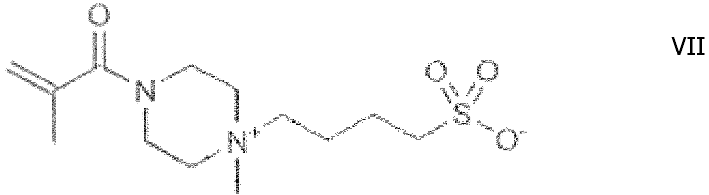

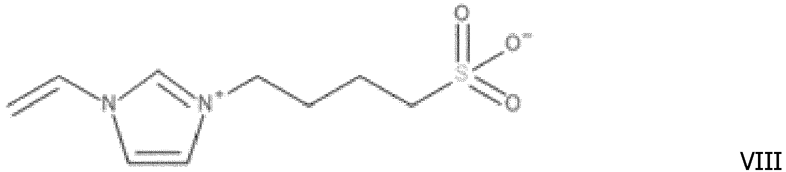

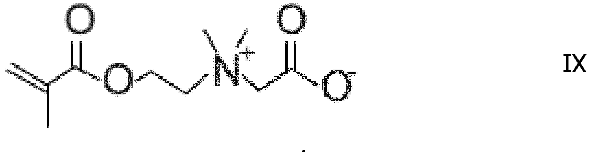

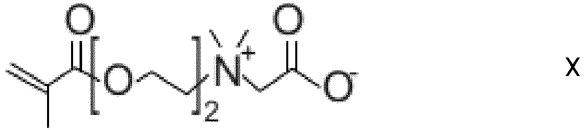

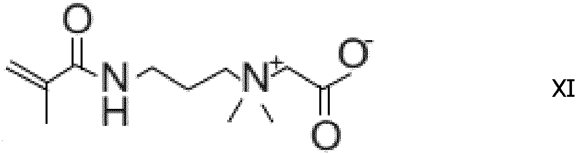

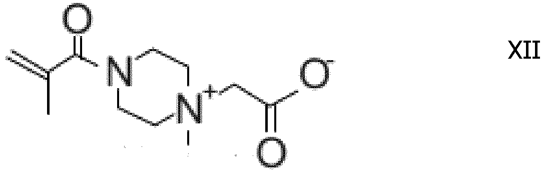

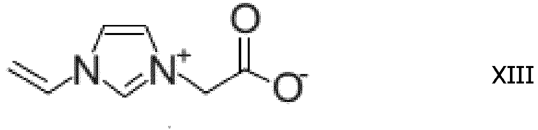

## Patentansprüche

1. Medizinische rohrförmige Vorrichtung, umfassend eine Körperstruktur mit einer länglichen hohlen Struktur, die sich von einem ersten Ende zu einem zweiten Ende der medizinischen rohrförmigen Vorrichtung erstreckt und eine Luminalfläche und eine Außenfläche aufweist, wobei die Körperstruktur eine kontinuierliche Matrix aus einer Wirtspolymermatrix aufweist und ein interpenetrierendes Polymernetzwerk (IPN) umfasst, das die Wirtspolymermatrix und mindestens eine Hydrogel-Gastpolymer-Domäne eines Gastpolymers umfasst, das eine Vielzahl von miteinander verbundenen Pfaden des Gastpolymers umfasst, das die Wirtspolymermatrix interpenetriert, wobei die Wirtspolymermatrix eine kovalent quervernetzte Elastomermatrix ist, wobei die mindestens eine Hydrogel-Polymer-Domäne eine Luminalflächen-Gastpolymer-Domäne umfasst, die mindestens einen Teil der Luminalfläche umfasst, wobei eine Vielzahl der Pfade des Gastpolymers zusammenfallen und mindestens einen Teil der Luminalfläche bilden und wobei die Luminalfläche zwitterionische Teile umfasst, wobei die zwitterionischen Teile kovalent an das Gastpolymer gebunden sind.

2. Rohrförmige Vorrichtung nach Anspruch 1, wobei das Gastpolymer ein zwitterionisches Hydrogel umfasst, das ein quervernetztes Netzwerk aus polymerisierten Monomeren umfasst, das eine oder mehrere Arten von Monomeren umfasst, wobei die zwitterionischen Monomere vorzugsweise mindestens eines von Sulfobetainen, Carbobetainen, Phosphobetainen, Phosphocholinen oder beliebigen Kombinationen davon umfassen.

3. Rohrförmige Vorrichtung nach einem der vorstehenden Ansprüche, wobei die Außenfläche eine Fläche mindestens teilweise des Gastpolymers umfasst, wobei eine Vielzahl der Pfade des Gastpolymers zusammenfallen und mindestens einen Teil der Außenfläche bilden, und wobei die Außenfläche zwitterionische Teile umfasst, wobei die zwitterionischen Teile vorzugsweise kovalent an das Hydrogel-(Gast-)Polymer gebunden sind.

4. Rohrförmige Vorrichtung nach einem der vorstehenden Ansprüche, wobei das Luminal mindestens ein kovalent gebundenes Arzneimittel umfasst, das ein Thrombose deaktivierendes Arzneimittel bzw. Thrombose deaktivierende Arzneimittel umfasst, wobei das Thrombose deaktivierende Arzneimittel beziehungsweise die Thrombose deaktivierenden Arzneimittel vorzugsweise Heparin, EDTA, Antibiotika, Citrat und/oder eine beliebige Kombination umfassen, die eines der genannten Thrombose deaktivierenden Arzneimittel umfasst.

5. Rohrförmige Vorrichtung nach einem der vorstehenden Ansprüche, wobei die Körperstruktur ein freisetzbares Arzneimittel umfasst, das ausgewählt ist aus einem antiproliferativen Arzneimittel, wie zum Beispiel Paclitaxel und/oder Rapamycin, das mindestens über die Luminalfläche freisetzbar ist, und einem anti-infektiösen Arzneimittel, wie zum Beispiel Rifampicin und/oder Minocyclin, das mindestens über die Außenfläche freisetzbar ist.

6. Rohrförmige Vorrichtung nach einem der vorstehenden Ansprüche, wobei das Wirtspolymer ein quervernetztes Elastomer umfasst, das vorzugsweise aus einem thermoplastischen Elastomer (TPE), einem Polyolefinelastomer (POE), einem Polyurethan (PU), einem Gummi, einem thermoplastischen Polyurethan (TPU), einem Silikonelastomer oder beliebigen Kombinationen, die eines der vorgenannten Elastomere umfassen, ausgewählt ist, wobei das Wirtspolymer vorzugsweise Silikonelastomer (Polysiloxane) umfasst.

7. Rohrförmige Vorrichtung nach einem der vorstehenden Ansprüche, wobei das Wirtspolymer eine Shore-A-Härte von etwa 15 bis etwa 70, eine Reißfestigkeit von mindestens etwa 25 kN/m und eine Spannung bei 200 % Dehnung von mindestens etwa 0,3 MPa, wie zum Beispiel mindestens etwa 0,4 MPa, wie zum Beispiel mindestens etwa 0,5 MPa, wie zum Beispiel mindestens etwa 0,6 MPa, wie zum Beispiel bis zu etwa 3 MPa, wie zum Beispiel bis zu etwa 2 MPa, wie zum Beispiel bis zu etwa 1 MPa aufweist.

8. Rohrförmige Vorrichtung nach einem der vorstehenden Ansprüche, wobei das Gastpolymer quervernetzt ist und aus Monomeren polymerisiert ist, die mindestens ein Zwitterion und mindestens eines von PHEMA, PEGMEA oder Vinylimidazolbutylsulfonat umfassen, wobei das Hydrogel-Gastpolymer vorzugsweise ein Copolymer umfasst, das aus Monomeren polymerisiert ist, die PHEMA, PEGMEA, Vinylimidazolbutansulfonat und/oder mindestens eines von Sulfobetanin umfassen.

9. Rohrförmige Vorrichtung nach einem der vorstehenden Ansprüche, wobei das Hydrogel-Gastpolymer ein Copolymer umfasst, das aus Monomeren polymerisiert ist, die mindestens ein zwitterionisches Monomer umfassen, das aus einem Sulfobetain, einem Carbobetain, einem Phosphobetain oder einem Phosphocholin ausgewählt ist, und/oder wobei das Hydrogel-Gastpolymer ein Copolymer umfasst, das aus Monomeren polymerisiert ist, die ein photoaktives Monomer umfassen, wie zum Beispiel ein Spiropyranmonomer, wie zum Beispiel ein Spiropyranacrylat.

10. Rohrförmige Vorrichtung nach einem der vorstehenden Ansprüche, wobei die mindestens eine Gastpolymer-Domäne eine gerinnungsfördernde Domäne und/oder eine zellproliferationsfördernde Domäne umfasst, die sich an der Außenfläche der Körperstruktur befindet und/oder an einer Zwischen-Gastpolymer-Domäne befindet, die nicht die Luminalfläche oder die Außenfläche umfasst.

11. Rohrförmige Vorrichtung nach einem der vorstehenden Ansprüche, wobei das Wirtspolymer zwei oder mehr Gastpolymer-Domänen umfasst, wobei die zwei oder mehr Gastpolymer-Domänen eine Luminalflächen-Gastpolymer-Domäne, die mindestens einen Teil der Luminalfläche umfasst, und eine Außenflächen-Gastpolymer-Domäne, die mindestens einen Teil der Außenfläche umfasst, umfassen, wobei die zwei oder mehr Gastpolymer-Domänen durch einen Polymerabschnitt getrennt sind, der im Wesentlichen frei von Gastpolymer ist, wobei der Polymerabschnitt vorzugsweise ein Abschnitt der Wirtspolymer-Matrix ist, der im Wesentlichen frei von interpenetrierendem Gastpolymer ist.

12. Rohrförmige Vorrichtung nach einem der vorstehenden Ansprüche, wobei die Körperstruktur eine geschichtete Körperstruktur ist, die zwei oder mehr Schichten umfasst, wobei mindestens eine der Schichten das IPN umfasst oder daraus besteht, wobei mindestens eine Schicht der Körperstruktur eine Polymerschicht ohne ein IPN ist, wie zum Beispiel eine Polymerschicht, die Polytetrafluorethylen (PTFE), expandiertes PTFE, Polyethylenterephthalat (PET), Polyurethan (PU) und/oder Silikonelastomer umfasst oder daraus besteht, wobei vorzugsweise die mindestens zwei Schichten der zwei oder mehr Schichten durch chemische Bindungen (Pfropfung) zwischen zwei Flächen miteinander verbunden sind und/oder mindestens zwei Schichten der zwei oder mehr Schichten miteinander verankert sind, wobei die Verankerung der mindestens zwei Schichten vorzugsweise eine lokale Klebeverbindung, Nähte, Klammern, Clips und/oder Nadellöcher umfasst.

13. Rohrförmige Vorrichtung nach einem der vorstehenden Ansprüche, wobei die rohrförmige Vorrichtung eine rohrförmige Stützstruktur umfasst, die sich konzentrisch zu der Körperstruktur befindet, wobei die Stützstruktur einen spiralförmigen Draht und/oder eine Manschette umfasst, die eng an der Außenfläche der Körperstruktur anliegt, und wobei die Stützstruktur aus einem Polymermaterial besteht, das Kollagen, Nylon, Polytetrafluorethylen (PTFE), expandiertes PTFE, Polyethylenterephthalat (PET), Polyurethan (PU) und/oder Silikonelastomer umfasst.

14. Rohrförmige Vorrichtung nach Anspruch 13, wobei die Manschette ein IPN-Material umfasst, das mit einem oder mehreren Arzneimitteln, wie zum Beispiel einem Wachstumshormon, antifibrinolytischem(n) Arzneimittel(n), gerinnungsförderndem(n) Arzneimittel(n) und/oder zellproliferationsförderndem(n) Arzneimittel(n), beladen ist.

15. Rohrförmige Vorrichtung nach einem der vorstehenden Ansprüche, wobei die Körperstruktur einen Innendurchmesser von etwa 2 mm bis etwa 6 cm, eine Nachgiebigkeit (% Innendurchmesseränderung pro mmHg × 10⁻² in dem Bereich von 80-200 mmHg) von etwa 1 bis etwa 10 und eine Wanddicke von bis zu etwa 1 cm, wie zum Beispiel von etwa 0,01 mm bis etwa 3 mm, wie zum Beispiel von etwa 0,5 mm bis etwa 1 mm aufweist.

16. Rohrförmige Vorrichtung nach einem der vorstehenden Ansprüche, wobei die medizinische rohrförmige Vorrichtung eine implantierbare rohrförmige Vorrichtung ist, wie zum Beispiel ein Dialysegraft, ein Stent, ein Gefäßgraft, ein Stentgraft oder eine Thoraflex-Hybridvorrichtung.

17. Rohrförmige Vorrichtung nach einem der vorstehenden Ansprüche, wobei die medizinische rohrförmige Vorrichtung eine Dialysesonde, eine Ernährungssonde, wie zum Beispiel eine Gastrostomiesonde, ein Venenkatheter, wie zum Beispiel ein zentraler Venenkatheter (CVC-Leitung) oder ein peripher eingeführter zentraler Venenkatheter (PICC-Leitung) ist.

18. Rohrförmige Vorrichtung nach einem der vorstehenden Ansprüche 2-17, wobei das mindestens eine zwitterionische Monomer ausgewählt ist aus zwitterionischen Monomeren mit der Formel I - XIII.

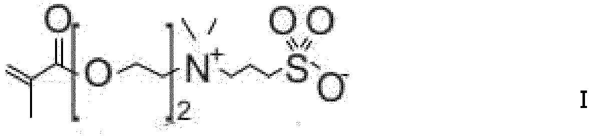

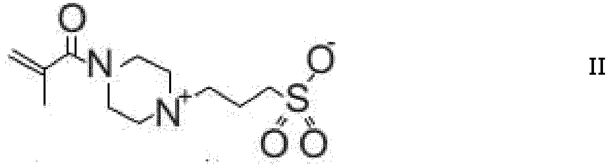

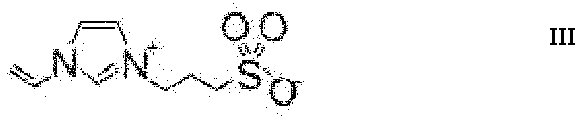

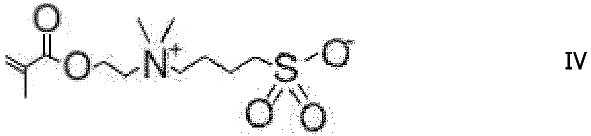

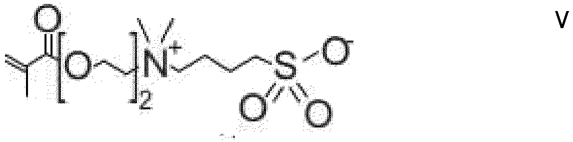

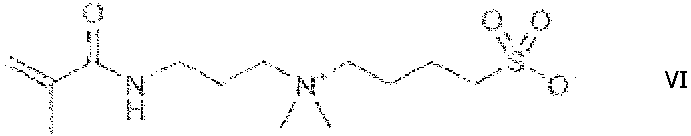

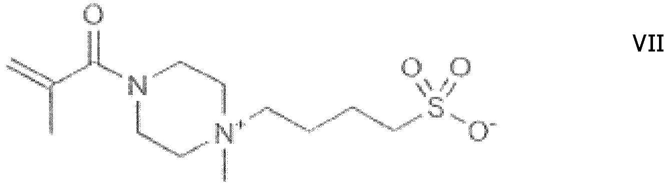

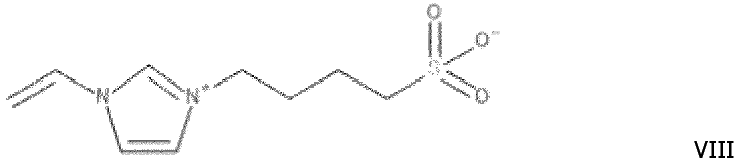

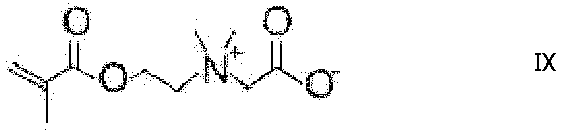

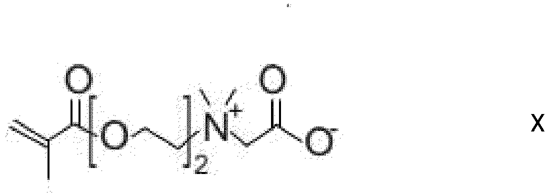

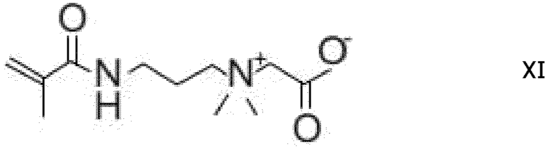

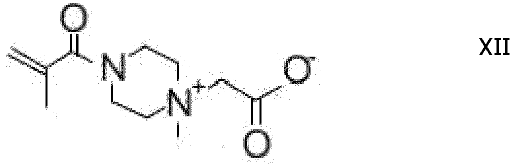

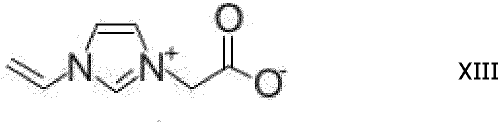

## Revendications

1. Dispositif tubulaire médical comprenant une structure de corps présentant une structure creuse allongée s'étendant d'une première extrémité à une seconde extrémité du dispositif tubulaire médical et présentant une surface luminale et une surface externe, dans lequel ladite structure de corps comporte une matrice continue constituée d'une matrice de polymère hôte et comprend un réseau de polymère s'interpénétrant (RPI) comprenant la matrice de polymère hôte et au moins un domaine de polymère en hydrogel inséré constitué d'un polymère inséré comprenant une pluralité de voies interconnectées du polymère inséré, qui s'interpénètrent dans la matrice de polymère hôte, dans lequel ladite matrice de polymère hôte est une matrice d'élastomère réticulée par liaison covalente, dans lequel ledit au moins un domaine de polymère en hydrogel comprend un domaine de polymère inséré de surface luminale constituant au moins une partie de la surface luminale, dans lequel une pluralité des voies du polymère inséré coïncident et forment au moins une partie de ladite surface luminale et dans lequel ladite surface luminale comprend des fractions zwitterioniques, lesdites fractions zwitterioniques étant liées par liaison covalente audit polymère inséré.

2. Dispositif tubulaire selon la revendication 1, dans lequel ledit polymère inséré comprend un hydrogel zwitterionique comprenant un réseau réticulé de monomères polymérisés consistant en un ou plusieurs types de monomères, de préférence les monomères zwitterioniques comprennent au moins une parmi les sulfobétaïnes, carbobétaïnes, phosphobétaïnes, phosphocholines ou toutes associations de celles-ci.

3. Dispositif tubulaire selon l'une quelconque des revendications précédentes, dans lequel la surface externe comprend une surface constituée au moins partiellement dudit polymère inséré où une pluralité des voies du polymère inséré coïncident et forment au moins une partie de ladite surface externe, et dans lequel ladite surface externe comprend des fractions zwitterioniques, lesdites fractions zwitterioniques étant de préférence liées par liaison covalente au dit polymère en hydrogel (inséré).

4. Dispositif tubulaire selon l'une quelconque des revendications précédentes, dans lequel la surface luminale comprend au moins un médicament lié par liaison covalente, comprenant un/des médicament(s) antithrombotique(s), le(s)dit(s) médicament(s) antithrombotique(s) comprenant de préférence l'héparine, l'EDTA, les antibiotiques, le citrate et/ou toute association comprenant l'un des médicaments antithrombotiques cités.

5. Dispositif tubulaire selon l'une quelconque des revendications précédentes, dans lequel ladite structure de corps comprend un médicament libérable choisi parmi un médicament antiprolifératif, tel que le paclitaxel et/ou la rapamycine libérable au moins via ladite surface luminale et un médicament anti-infectieux tel que la rifampicine et/ou la minocycline, libérable via au moins ladite surface externe.

6. Dispositif tubulaire selon l'une quelconque des revendications précédentes, dans lequel le polymère hôte comprend un élastomère réticulé, choisi de préférence parmi un élastomère thermoplastique (TPE), un élastomère polyoléfinique (POE), un polyuréthane (PU), un caoutchouc, un polyuréthane thermoplastique (TPU), un élastomère silicone ou toutes associations comprenant l'un des élastomères précités, de préférence le polymère hôte comprend un élastomère silicone (polysiloxanes).

7. Dispositif tubulaire selon l'une quelconque des revendications précédentes, dans lequel le polymère hôte a une dureté Shore A d'environ 15 à environ 70, une résistance à la déchirure d'au moins environ 25 kN/m et une contrainte à l'allongement à 200 % d'au moins environ 0,3 MPa, comme d'au moins environ 0,4 MPa, comme d'au moins environ 0,5 MPa, comme d'au moins environ 0,6 MPa, comme d'au maximum environ 3 MPa, comme d'au maximum environ 2 MPa, comme d'au maximum environ 1 MPa.

8. Dispositif tubulaire selon l'une quelconque des revendications précédentes, dans lequel le polymère inséré est réticulé, et est polymérisé à partir de monomères comprenant au moins un zwitterion et au moins un parmi le PHEMA, le PEGMEA ou le vinylimidazole butylsulfonate, de préférence le polymère en hydrogel inséré comprend un copolymère polymérisé à partir de monomères comprenant le PHEMA, le PEGMEA, le vinylimidazole butanesulfonate et/ou au moins une choisie parmi les sulfobétaïnes.

9. Dispositif tubulaire selon l'une quelconque des revendications précédentes, dans lequel le polymère en hydrogel inséré comprend un copolymère polymérisé à partir de monomères comprenant au moins un monomère zwitterionique, choisi parmi une sulfobétaïne, une carbobétaïne, une phosphobétaïne, ou une phosphocholine et/ou dans lequel le polymère en hydrogel inséré comprend un copolymère polymérisé à partir de monomères comprenant un monomère photoactif, tel qu'un monomère spiropyrane, tel qu'un spiropyrane acrylate.

10. Dispositif tubulaire selon l'une quelconque des revendications précédentes, dans lequel ledit au moins un domaine de polymère inséré comprend un domaine favorisant la coagulation et/ou un domaine favorisant la prolifération cellulaire localisé(s) à la surface externe de la structure de corps et/ou localisé(s) dans un domaine intermédiaire de polymère inséré, qui ne constitue pas la surface luminale ou la surface externe.

11. Dispositif tubulaire selon l'une quelconque des revendications précédentes, dans lequel le polymère hôte comprend deux ou plus de deux domaines de polymère inséré, dans lequel les deux ou plus de deux domaines de polymère inséré comprennent un domaine de polymère inséré de surface luminale constituant au moins une partie de la surface luminale et un domaine de polymère inséré de surface externe constituant au moins une partie de la surface externe, les deux ou plus de deux domaines de polymère inséré étant séparés par une partie polymère essentiellement exempte de polymère inséré, ladite partie polymère étant de préférence une partie de ladite matrice de polymère hôte, qui est essentiellement exempte de polymère inséré s'interpénétrant.

12. Dispositif tubulaire selon l'une quelconque des revendications précédentes, dans lequel la structure de corps est une structure de corps stratifiée comprenant deux ou plus de deux couches, dans lequel au moins une desdites couche comprend ou consiste en le RPI, dans lequel au moins une couche de la structure de corps est une couche de polymère sans un RPI, telle qu'une couche de polymère comprenant ou consistant en du polytétrafluoroéthylène (PTFE), PTFE expansé, poly(éthylène téréphtalate (PET), polyuréthane (PU) et/ou de l'élastomère silicone, de préférence lesdites au moins deux couches des deux ou plus de deux couches sont liées interfacialement entre elles par des liaisons chimiques (greffage) et/ou au moins deux couches des deux ou plus de deux couches sont ancrées l'une à l'autre, ledit ancrage desdites au moins deux couches comprend de préférence une liaison adhésive locale, des sutures, agrafes, clips et/ou trous d'aiguille.

13. Dispositif tubulaire selon l'une quelconque des revendications précédentes, dans lequel le dispositif tubulaire comprend une structure tubulaire de soutien disposée concentriquement avec la structure de corps, dans lequel la structure de soutien comprend un fil métallique hélicoïdal et/ou un manchon étroitement adapté à la surface externe de la structure de corps et dans lequel la structure de soutien est constituée d'une matière polymère comprenant le collagène, le nylon, le polytétrafluoroéthylène (PTFE), le PTFE expansé, le poly(éthylène téréphtalate) (PET), le polyuréthane (PU) et/ou un élastomère silicone.

14. Dispositif tubulaire selon la revendication 13, dans lequel le manchon comprend une matière RPI chargée avec un ou plusieurs médicaments, tel(s) qu'une hormone de croissance, un/des médicament(s) antifibrinolytique(s), médicament(s) favorisant la coagulation et/ou médicament(s) favorisant la prolifération cellulaire.

15. Dispositif tubulaire selon l'une quelconque des revendications précédentes, dans lequel la structure de corps a un diamètre interne d'environ 2 mm à environ 6 cm, une compliance (% de variation du diamètre interne par mm Hg × 10⁻² dans la plage de 80-200 mm Hg) d'environ 1 à environ 10 et une épaisseur de paroi de jusqu'à environ 1 cm, comme d'environ 0,01 mm à environ 3 mm, comme d'environ 0,5 mm à environ 1 mm.

16. Dispositif tubulaire selon l'une quelconque des revendications précédentes, dans lequel le dispositif tubulaire médical est un dispositif tubulaire implantable, tel qu'une greffe pour dialyse, un stent, une greffe vasculaire, un stent couvert ou un dispositif hybride Thoraflex.

17. Dispositif tubulaire selon l'une quelconque des revendications précédentes, dans lequel le dispositif tubulaire médical est un tube de dialyse, un tube d'alimentation, tel qu'un tube de gastrotomie, un cathéter veineux, tel qu'un cathéter veineux central (CVC line) ou un cathéter veineux central inséré par voie périphérique (PICC line).

18. Dispositif tubulaire selon l'une quelconque des revendications 2-17 précédentes, dans lequel ledit au moins un monomère zwitterionique est choisi parmi les monomères zwitterioniques de formules I - XIII.
